# FASCICULE DE BREVET EUROPEEN

(11) **EP 0 967 233 B1**
(45) Date de publication et mention de la délivrance du brevet: **01.03.2006**
(21) Numéro de dépôt: 99112241.7
(22) Date de dépôt: 25.06.1999
(51) Int. Cl.: C08F 20/40, C08F 12/14, C08F 20/28, C08F 20/36, C08G 65/14, C08G 65/22, H01M 10/40, H01M 6/18

(54) **Matériau à conduction ionique**
Material mit ionischer Leitfähigkeit
Ion conductive material

(30) Priorité: 10.07.1998 CA 2243103; 25.06.1998 CA 2242017
(43) Date de publication de la demande: 29.12.1999
(73) Titulaire: HYDRO-QUEBEC, Montréal, Québec H2Z 1A4 (CA)
(72) Inventeur: Michot, Christophe, 69008 Lyon (FR); Gauthier, Michel, Laprairie, Quebec J5R 1E6 (CA); Vallee, Alain, Ouest, Montreal, Quebec H2Z 1A4 (CA); Harvey, Paul-Etienne, Ouest, Montreal, Quebec H2Z 1A4 (CA); Armand, Michel, Montreal, Quebec H3T 1N2 (CA)
(74) Mandataire: Sueur, Yvette

(56) Documents cités:
- EP-A- 0 421 230
- EP-A- 0 657 485
- GB-A- 836 046
- US-A- 5 146 005
- US-A- 5 665 841

## Description

### DOMAINE DE L'INVENTION

La présente invention concerne un matériau à conduction ionique constitué par au moins un polymère obtenu par amorçage anionique et portant des fonctions pouvant être activées par amorçage cationique qui ne sont pas réactives en présence de réactifs amorçant les polymérisations anioniques et au moins un composé ionique. La présence de telles fonctions à amorce cationique permettent une réticulation efficace du polymère après mise en forme, ou en particulier sous forme de film mince. Il est ainsi possible d'obtenir des polymères possédant des propriétés très bien définies, en termes masse moléculaire et densité de réticulation.

### ART ANTÉRIEUR

On connaît principalement trois types de mécanisme de polymérisation, en l'occurrence anionique, cationique et radicalaire. Généralement, les monomères portant des fonctions de type double liaison conjuguée ou activée permettant la propagation d'espèces radicalaires sont les plus utilisées. Il est par contre difficile de contrôler les masses moléculaires obtenues, d'autant plus que les radicaux sont inactivés par l'oxygène. Plus récemment, les monomères portant des fonctions de type éther vinylique CH₂=CHO- ayant une grande réactivité en polymérisation cationique ont été commercialisés. La polymérisation cationique, bien que très rapide, donne difficilement des masses moléculaires élevées principalement du fait que les carbocations permettant la propagation de la polymérisation sont sensibles à l'eau ou autres nucléophiles présents dans le milieu réactionnel.

Les compositions les plus utilisées pour la fabrication de films, vernis ou encres combinent en général des monomères de type époxyde avec des monomères portant une ou plusieurs fonctions éther vinylique et qui sont polymérisés par des catalyseurs cationiques. La polymérisation des époxydes et celle des éthers vinylique CH₂=CHO- se propageant à des vitesses très différentes, les solides macromoléculaires obtenus sont habituellement constitués de réseaux interpénétrés correspondant à chaque type de monomères. Le degré de polymérisation et la densité de réticulation effective de tels systèmes sont donc difficilement contrôlables. Les propriétés mécaniques sont plus dépendantes de la rigidité des chaînes ou de la présence de fonctions OH donnant des liaisons hydrogène fortes. Ces compositions, en particulier celles auxquelles sont ajoutés les diéthers vinyliques des glycols ou triols, permettent néanmoins de minimiser la consommation de solvants volatils du fait de la faible viscosité des monomères correspondants (appelés "reactive diluents"). Les éthers vinyliques présentent à ce point de vue une faible toxicité.

La notion de "réactive diluent" est aussi utilisée des mélanges de monomères de type acrylique (radicalaire) avec des monomères de type éthers vinyliques, en présence d'amorceurs radicalaires ajoutés à des amorceurs cationiques. Il est néanmoins tout aussi difficile par cette technique d'assurer un contrôle régulier du taux de polymérisation/réticulation du fait de la sensibilité à l'oxygène de la polymérisation radicalaire. Ce problème se pose particulièrement pour les films minces exposant une surface de contact importante à l'air. Des monomères polyfonctionnels contenant une fonction éther vinylique ont été décrits dans US 5,605,941. Ces composés sont destinés à obtenir des résines réticulées ayant une température de transition vitreuse élevée par un procédé en une seule étape faisant intervenir des réseaux interpénétrés de type (cationique + cationique) ou (cationique + radicalaire).

La polymérisation anionique présente de nombreux avantages en termes de contrôle précis de la masse moléculaire, en particulier une distribution des masses M_{w}/Mₙ étroite. Cette méthode reste celle de choix pour la préparation de polymères de masse prédéterminée et pour l'élaboration de polymères à bloc, de type AB, ABA, ou ramifiés. Cependant, les amorceurs ainsi que les espèces anioniques permettant la propagation sont très réactifs. Ces espèces sont soit des organométalliques, soit des alkoxydes de métaux alcalins qui réagissent sur la plupart des fonctions organiques portées par les monomères, en particulier celles qui permettraient une réticulation ultérieure facile, telle que les fonctions contenant des époxydes, alcools ou amines, ou des doubles liaisons activées par une ou des doubles liaisons conjugués, un noyau aromatique ou groupement attracteur d'électrons tel que C=O, C≡N. Les conditions d'exclusion d'eau ou autre nucléophile lors de la polymérisation font que ce type de polymère est préparé dans des unités dédiées, et non pas au moment de l'utilisation ou de la mise en forme.

On connaît de plus les polymères possédant des fonctions éther en concentration élevée, en général de 40 à 100% molaire, en particulier contenant les unités -[(CH₂H(R)O]ₙ- où 4 ≤ n ≤ 2 x 10⁴ et R est H ou un groupement alkyle simple de 1 à 4 atomes de carbone, ou bien un groupement polymérisable tel que le groupement allyloxyméthyl CH=CHCH₂OCH₂-. Les copolymères, en particulier ceux dans lesquels R est principalement H, possèdent la propriété de dissoudre certains sels, métalliques ou d'onium (ammonium, amidinium, guanidinium) pour former des solutions solides conductrices. Les sels de lithium sont particulièrement utiles pour former des électrolytes utilisables dans les batteries primaires ou secondaires, les super-capacités ou les dispositifs de modulation de la lumière dits "électrochromes". L'environnement dans lequel ces matériaux fonctionnent, en particulier en contact avec des éléments très réducteurs, tel que le lithium métallique, ses alliages ou les solutions solides de ce métal dans les différentes formes du carbone, dont le graphite et les cokes, exige une grande stabilité des liaisons constitutives du polymère, principalement limitées à des liaisons CH et C-O des fonctions éther. La faible conductivité intrinsèque de ces matériaux amène à les mettre en oeuvre en films minces ayant néanmoins de bonnes tenues mécaniques. Ceci est obtenu par l'emploi des masses moléculaires élevées ou encore plus commodément par un processus de réticulation. Cette dernière méthode a par contre le désavantage d'augmenter la température de transition vitreuse (T_{g}) du réseau, qui est le paramètre le plus important pour déterminer la conductivité. De plus, les fonctions allyloxyméthyl introduisant des fonctions permettant la réticulation, en particulier avec le monomère allyl-glycidyl éther (AGE), qui sont résistantes à l'action des catalyseurs permettant la polymérisation anionique, ne sont que peu actives pour la formation ultérieure de noeuds de réticulation, et il est impossible de contrôler exactement la densité de réticulation des matériaux contenant ce monomère. Il est difficile de faire participer plus de 50% des double liaisons à la réticulation.

Des polymères préparés à partir d'éthers vinyliques d'oligo(oxyde d'éthylène) ont été proposés comme électrolytes solides, par exemple dans US 4,886,716, US 5,064,548, US 5,173,205, US 5,264,307, US 5,411,819 et US 5,501,920 et présentent des propriétés de conductivité acceptables. La préparation des monomères correspondants est cependant délicate. Ces matériaux avant réticulation sont de faible masse moléculaire, tout comme pour la plupart des polymérisations cationiques. En effet, les monomères sont très hygroscopiques, et donc difficiles à purifier. En outre, les propriétés mécaniques des polymères sont médiocres du fait de l'absence d'enchevêtrement, phénomène propre aux polymères à chaînes latérales. Par l'utilisation de monomères polyfonctionnels de type diéther vinylique, il est possible d'obtenir des produits réticulés, mais il est par contre impossible de séparer l'étape de polymérisation de celle de réticulation par processus cationique, ce qui fait en sorte que l'on a pratiquement aucun contrôle sur le processus d'obtention du polymère réticulé.

### SOMMAIRE DE L'INVENTION

La présente invention concerne un matériau à conduction ionique constitué par au moins un polymère et un au moins composé ionique, caractérisé en ce que :
- le polymère est un polymère réticulable préparé par polymérisation anionique d'un monomère répondant à la formule générale (A)ₙQ(Y)ₚ éventuellement avec un ou plusieurs autres monomères, dans laquelle:
   * Q représente une liaison, ou un radical organique de valence n + p non réactif vis-à-vis des agents initiant la polymérisation anionique ou cationique comprenant un radical alkyle, alkylaryle, arylalkyle comprenant optionnellement des substituants oxa ou aza, et comprenant de 1 à 30 atomes de carbone,
   * A représente un radical réactif en polymérisation anionique,
   * Y est un radical réactif en polymérisation cationique et non réactif vis-à-vis d'un agent initiant la polymérisation anionique et représente la fonction réticulable dudit polymère réticulable,
   * n varie entre 1 et 3;
   * p varie entre 1 et 6, et
- le composé ionique est constitué par au moins un sel Mⁿ⁺Xₙ⁻, dans lequel Mⁿ⁺ représente un cation inorganique, organique ou organométallique quelconque de charge n, et X- est un anion monovalent, deux ou plusieurs anions X⁻ pouvant être liés ensemble par des chaînes covalentes ou pouvant appartenir à la chaîne d'un polymère.

Dans une mise en oeuvre préférentielle, A comprend

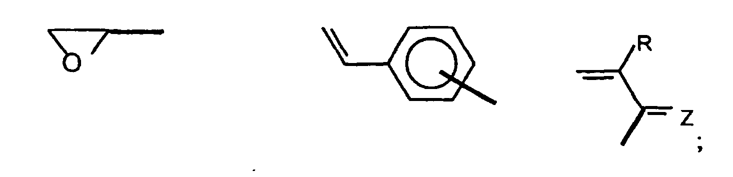

Y comprend 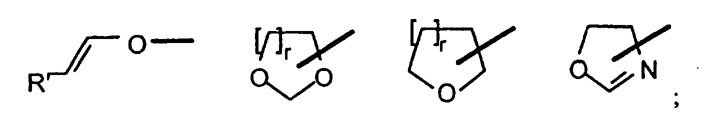
dans lesquels
Z représente O ou CH₂;
R représente H, un radical alkyle ou oxa-alkyle de 1 à 12 atomes de carbone, CN ou CH₂COOR¹ dans lequel R¹ est H ou un radical alkyle ou oxa-alkyle de 1 à 12 atomes de carbone;
R' représente H ou un radical alkyle de 1 à 12 atomes de carbone; et
r varie entre 1 et 6.

Les polymères de l'invention sont capables de dissoudre des composés ioniques induisant une conductivité, pour la préparation d'électrolytes.

### DESCRIPTION DÉTAILLÉE DE L'INVENTION

La présente invention a pour objet des polymères obtenus par amorçage anionique et portant des fonctions pouvant être activées par amorçage cationique, et permettant une réticulation efficace du polymère après mise en forme, ou en particulier sous forme de film mince. Les fonctions actives en polymérisation cationique sont donc choisies pour leur stabilité vis-à-vis des réactifs permettant la polymérisation anionique. Il est ainsi possible d'obtenir des polymères possédant des propriétés très bien définies, en termes de masse moléculaire et de densité de réticulation, en mettant à profit les avantages cumulés de ces deux types de polymérisation. La réticulation est réalisée de façon efficace, et totalement indépendante en particulier de l'action de l'oxygène.

Font partie intégrale et sont un aspect important de l'invention, des polymères préparés par voie anionique et réticulés par voie cationique, capables de dissoudre des composés ioniques induisant une conductivité pour la préparation d'électrolytes solides. Tel que mentionné précédemment, la nécessité de pouvoir introduire des motifs permettant la réticulation sans compromettre la stabilité chimique ou électrochimique du système et sans augmentation importante de la température de transition vitreuse, est déterminante. Les polymères de l'invention répondent à ces critères par le choix de groupements actifs en polymérisation cationique et permettant une réticulation efficace et contrôlée.

Les monomères utilisés pour l'obtention d'un polymère selon l'invention sont définis par la formule générale:

(A)ₙQ(Y)ₚ

dans laquelle
Q représente une liaison, -CO- ou -SO₂-, un radical organique de valence n + p non réactif vis-à-vis des agents initiant la polymérisation anionique ou cationique, et de type alkyle, alkylearyle ou arylealkyle, possédant optionnellement des substituants oxa ou aza, et comprenant de 1 à 30 atomes de carbone;
A représente un radical réactif en polymérisation anionique;
Y représente un radical réactif en polymérisation cationique et non réactif vis-à-vis des agents initiant la polymérisation anionique;
n varie entre 1 et 3; et
p varie entre 1 et 6.

De façon préférentielle, n est égal à 1 sauf dans le cas où il est avantageux de disposer directement d'un matériau réticulé et dont il est souhaitable de poursuivre la réticulation par mode cationique. Lorsque n est égal à 2, deux fonctions portées par le même monomère polymérisent d'une façon concertée, comme pour celles amenant la formation de cycles sans créer de réticulation. Il peut aussi être intéressant de mélanger des monomères pour lesquels n = 1 à une faible fraction, par exemple de 0.1 à 10 %, de monomère polyfonctionnel de manière à augmenter la masse moléculaire par création de branchements compensant la terminaison naturelle des chaînes.

Les polymères de l'invention sont préparés par polymérisation anionique de monomères tels que définis plus haut dans lesquels A comprend préférentiellement: 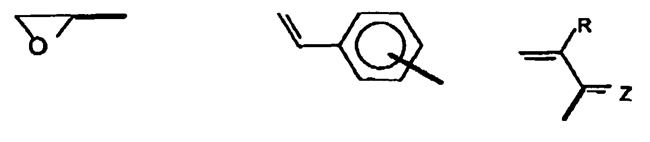 dans lesquels
Z = O ou CH₂; et
R représente H, un radical alkyle ou oxa-alkyle de 1 à 12 atomes de carbone, CN ou CH₂COOR¹ dans lequel R¹ est II ou un radical alkyle ou oxa-alkyle de 1 à 12 atomes de carbone.

Dans ces mêmes monomères, Y comprend préférentiellement: 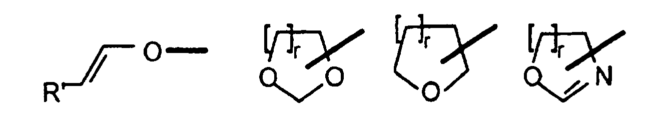
dans lesquels
R' représente H ou un radical alkyle de 1 à 12 atomes de carbone; et
r varie entre 1 et 6.

La durée de vie des espèces cationiques permettant la propagation de la polymérisation/réticulation étant particulièrement longue comparée aux espèces radicalaires, la réaction peut se poursuivre après initiation par un amorceur de polymérisation cationique, jusqu'à consommation complète des groupements Y. En particulier, une exposition très brève à la chaleur ou à une radiation actinique suffit pour initier les espèces réactives, et la polymérisation peut se poursuivre même en l'absence de l'agent activant, surtout grâce à l'absence de réaction de terminaison par l'oxygène de l'air.

Les amorceurs de type anionique utilisables comprennent préférentiellement les organométalliques, les amidures, les alkoxydes, les bases fortes dérivées des dialkylaminophosphines. Les organométalliques préférentiels comprennent les alkyllithium, tel que le butyllithium (primaire, secondaire ou tertaire), l'hexyllithium, les dérivés du lithium, sodium ou potassium du 1,1'-diphényléthylène, du tétraphényléthylène, du naphtalène, du biphényle et de la benzophénone. Les amidures préférentiels comprennent NaNH₂, Ca(NH₂)₂, leurs produits d'addition avec les époxydes, les dialkylamidures comme le di-isopropylamidure de lithium. Les alcoxydes préférentiels comprennent les méthoxydes, éthoxydes, butoxydes (primaire, secondaire, tertiaire) ou *tert*-amyloxyde des métaux alcalins, les alcoxydes des alcools linéaires de 8 à 18 atomes de carbone, les polyéthylènes monoalcool de masse comprise entre 400 et 800, les alcoxydes des alcools polyhydriques, tels que le glycol, le glycérol, les oligoéthylènes glycol, le sorbitol, le pentaérythritol, le triméthylolpropane, le bis-triméthyloldiéthyléther, le bisphénol A et leurs dérivés polyéthoxylé. Les bases fortes préférentielles comprennent les dérivés des dialkylaminophosphines comme le 1-*tert*-butyl-4,4,4,-tris[diméthylamino-2,2-bis(trisdiméthylamino)-phosphoranylidèneamino]-2λ5-4λ5-catenadi(phosphazène), communément appelée "phosphazène base P4-*t*-bu". D'une manière générale, les dérivés organométalliques et les dialkylamidures, en particulier les dérivés du lithium, sont préférés pour amorcer la polymérisation des doubles liaisons activées par d'autres doubles liaisons conjuguées C=C ou C=O, tels que les dérivés du butadiène, du styrène, de l'acide acrylique ou méthacrylique. Pour la polymérisation des époxydes, les dérivés des métaux alcalins tel que le sodium et plus particulièrement le potassium des alcools mono- ou polyhydroxyliques amenant la formation de chaînes linéaires ou ramifiées, sont préférées. Les dialkylaminophosphines sont de leur côté réactives pour les acrylates ou les époxydes. L'activité des dérivés organométalliques, amidures ou alcoxydes, peut être augmentée en présence de molécules susceptibles de solvater fortement les ions alcalins, par exemple le THF, les éthers di-alkyliques des oligoéthylènes glycols ayant de 2 à 16 atomes de carbone et pouvant aussi être utilisés comme solvant. Les peralkyl(polyéthylèneimines) de 2 à 8 atomes d'azote, en particulier la tétraméthyléthylène diamine (TMDA), la pentaméthyldiéthylènediamine, et la tris(2-diméthylaminoéthylamine) ont des propriétés activantes particulièrement marquées.

Les polymères de l'invention obtenus après l'étape de polymérisation anionique initiale sont réticulés par un processus cationique en utilisant les groupements du monomères prévues à cet effet. D'une manière générale, les catalyseurs permettant la propagation de la réaction de polymérisation/réticulation sont des acides de Lewis ou de Brønsted. Les acides de Lewis parmi les plus réactifs comprennent les dérivés de l'aluminium, du bore, du zinc de type B(Hal)₃, Al(Hal)₃, Zn(Hal)₂, dans lesquels Hal est un halogène ou pseudohalogène, un groupement alkyle, aryle, ou des halogénures de zinc. Les acides de Brønsted sont de leur côté susceptibles de donner un cation dont la charge de surface est supérieure à 3 x 10⁻¹⁹ coulombs/Å². Les autres cations qui correspondent au critère de charge peuvent d'une manière non limitée comprendre Li⁺, Mg²⁺, Ca²⁺, Zn²⁺, Sn²⁺, Al³⁻ etc. Les dérivés parmi les plus réactifs sont des acides forts dont les anions X⁻ sont faiblement basiques ou faiblement nucléophiles. Des exemples de tels anions X⁻ sont AlCl₄⁻, BF₄⁻, PF₆⁻, AsF₆⁻, SbF₆⁻, TeOF₅⁻, R²SO₃⁻ ou B(R²)₄⁻ dans lequel R² est le fluor, un groupement organique alkyle ou aryle, halogéné ou non; les imidures (R²SO₂)₂N⁻; les méthylures (R²SO₂)₂C(R³)⁻, (R²SO₂)₃C⁻ où R³ est H ou R². Les acides peuvent être directement ajoutés au polymère ou être sous forme latente. Les sels de bases faibles telles que les bases azotés ou les éthers, conviennent pour cet usage, et la forme acide est libérée par chauffage, de préférence à des températures comprises entre 40 et 180°C. Il est aussi possible de libérer l'acide thermiquement à partir d'un sel de diazonium RN=N⁺X⁻ se décomposant pour donner l'acide HX et de l'azote par l'arrachement de protons au solvant ou monomère.

Les esters d'acides correspondants à des anions non nucléophiles sont des amorceurs cationiques efficaces. Ces derniers comprennent les toluène-, fluoro-, méthane- et trifluorométhanesulfonates de méthyle, d'éthyle, de benzyle, les diesters du tétraméthylène -(CH₂)₄-.

D'une manière très avantageuse, les acides peuvent être libérés par action de radiations actiniques sur des composés labiles. Par radiation actinique, on entend les photons visibles ou UV, les radiations ionisantes, dont les rayons γ et les faisceaux d'électrons β. Les photoamorceurs cationiques, autrement dits photogénérateurs d'acide, comprennent les composés ioniques J⁺X⁻ dans lesquels X⁻ représente un anion tel que défini précédemment et J⁺ représente un cation de type diaryliodionium, diarylbromonium, triarylsulfonium, phénacyl-dialkylsulfonium, arène-métallocènium, arylesdiazonium, les groupements organiques étant optionnellement substitués. Deux ou plusieurs cations J⁺ peuvent être reliés entre eux ou J⁺ peut faire partie d'une unité récurrente d'une chaîne polymère. Une autre famille de photoamorceurs ou amorceurs thermiques comprend avantageusement les esters sulfoniques du 2-nitro, 2,4-dinitro ou 2,6-dinitrobenzyle, en particulier les toluènesulfonates du 2,4-dinitro ou 2,6-dinitrobenzyle. Ces amorceurs ne sont pas ioniques, et donc facilement miscibles avec les monomères et polymères peu ou non polaires.

Il existe d'autres amorceurs cationiques, en particulier des dérivés des sels de l'allyloxypyridinium qui, en présence de générateurs radicaux libres, activés thermiquement ou par un rayonnement actinique, libèrent des cations alkyle ou pyridyle. On peu citer l'hexafluoroantimonate de N-[2-éthoxycabonylallyloxy]-α-picolinium.

La technique de l'invention est particulièrement avantageuse pour la préparation de polymères sous forme de films, du fait de l'insensibilité du processus de polymérisation cationique à l'oxygène, en particulier en comparaison avec les techniques mettant en jeu des processus radicalaires. La portée du procédé n'est cependant pas limitée à ce type de mise en forme. Les polymères avant réticulation peuvent être façonnés en forme quelconque, après ajout d'un catalyseur latent de polymérisation cationique qui est activé soit par la chaleur soit par une radiation actinique pénétrante.

Il peut être intéressant de minimiser les contractions de volume inhérentes à la plupart des processus de polymérisation, y compris la réticulation. Les processus cationiques mettant en jeu des dérivés oxygénés des dioxolanes de type spiroorthoformates ou spiroorthocarbonates sont caractérisés par une augmentation de volume. Des monomères et polymères de l'invention portant de telles fonctions, qui peuvent être utilisés pour contrôler les variations de volume, sont exemplifiés par les formules suivantes:

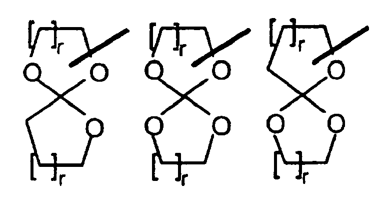

Un autre avantage que les groupements de réticulation cationique de l'invention apportent, outre le caractère contrôlable et complet de la réticulation, est la flexibilité du sous-réseau obtenu. Les fonctions résultant de la réticulation cationique portant des groupements de type : 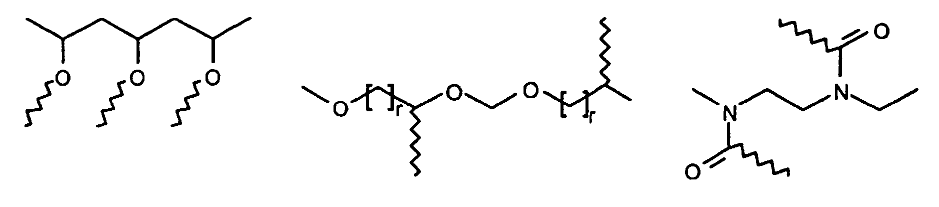 sont intrinsèquement flexibles, et les homopolymères correspondants ont des températures de transition vitreuse (T_{g}) basses. Par exemple, T_{g} = -31°C pour le polyvinylméthyl éther, alors que T_{g} = + 114°C pour le polyméthacrylate de méthyle. Ces caractéristiques peuvent être maintenues au niveau du sous-réseau cationique du polymère par le choix de liens flexibles reliant les groupements anioniques aux groupements cationiques. Les alkylène simples -(CH₂)ₙ- dans lesquels 2 ≤ n ≤ 12 et oxyalkylène -[CH₂OCH(R)]ₙ- dans lesquels 1 ≤ n ≤ 6, sont particulièrement préférées pour former les liens organiques divalents assurant la jonction entre les groupements anioniques et les groupements cationiques dans le monomère et/ou le polymère résultant.

Les polymères de la présente invention peuvent être des homo- ou copolymères statistiques incorporant des monomères à double fonctionnalité et un ou plusieurs autres monomères. Par exemple, on dénote Aₐ*-stat-*B_{b}*-stat-*C_{c}, un terpolymère contenant les monomères A, B et C dans des rapports molaires a, b, et c respectivement. Ils peuvent être des polymères à bloc, de type A*-bloc-*B ou A*-bloc-*B*-bloc-*A, A*-bloc-*B*-bloc-*C, chaque segment A, B ou C pouvant incorporer à des taux variés les monomères à double fonctionnalité. Dans une variante, seul A, B ou C incorpore sous forme d'homopolymère ou de copolymère au moins un monomère à double fonctionnalité. Il est entendu que les polymères de l'invention ne sont pas limités à trois monomères et que toute personne du métier peut apprécier les variations possibles offertes par le caractère vivant de la polymérisation anionique. Une autre possibilité est de former des polymères en étoile ou des dendrimères à partir d'amorceurs anioniques polyfonctionnels de type T(Aₐ*-stat*-B_{b-}*stat-*C_{c})ₜ, T(A-*bloc*-B)ₜ ou T(A-*bloc*-B-*bloc*-A)ₜ ou encore T(A-*bloc*-B-*bloc*-C)ₜ, T étant un radical polyfonctionnel de valence t, 2 ≤ t ≤ 10⁴, de préférence, 2 ≤ t ≤ 10 pour les structures étoiles, et pouvant avoir une valeur jusqu'à 10⁴ pour les dendrimères. Des polymères branchés peuvent être obtenus par l'utilisation de faibles taux d'un monomère possédant plus d'une fonctionnalité réactive en polymérisation anionique.

La masse des polymères avant réticulation est de préférence comprise entre 2 x 10³ et 60 x 10³ g/mol.

Un cas particulier de polymères selon l'invention concerne un copolymère réticulable obtenu à partir :
- d'un composé (A)ₙQ(Y)ₚ dans lequel A comprend un groupe

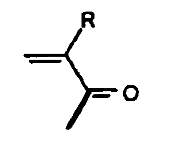

dans lequel R représente H, un radical alkyle ou oxa-alkyle de 1 à 12 atomes de carbone, CN on CH₂COOR¹ dans lequel R¹ est H ou un radical alkyle ou oxa-alkyle de 1 à 12 atomes de carbone ;
- d'un composé ayant une double liaison activée par un groupement carbonyle, choisi par exemple parmi les acrylates, les méthacrylates ou les itaconates de α-alkyl(oligoéthoxy)éthyle de formule générale: 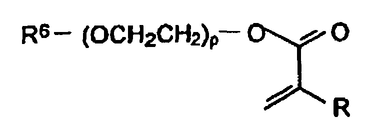
   dans laquelle
   - p varie entre 2 et 60;
   - R représente H, un radical alkyle ou oxa-alkyle de 1 à 12 atomes de carbone, CN ou CH₂COOR¹ dans lequel R¹ est H ou un radical alkyle ou oxa-alkyle de 1 à 12 atomes de carbone; et
   - R⁶ est un radical alkyle monovalent de 1 à 18 atomes de carbone, ou un radical aryle monovalent de 5 à 18 atomes de carbone.
- et d'au moins un autre monomère de type acrylate organique ou métallique.

L'étape de mise en forme et de réticulation peut être précédée ou simultanée à l'addition d'adjuvants divers, par exemple une dispersion de solides, sous forme de poudres, de lamelles ou de fibres, destinés à changer les propriétés rhéologiques et de produit lors de la mise en forme et/ou conférer des caractéristiques mécaniques améliorées au produit fini ainsi que des propriétés ignifuges. L'additif peut être organique ou inorganique, et posséder optionnellement des propriétés de conduction ionique ou électronique intrinsèques, des propriétés optiques, une constante diélectrique élevée, des propriétés piézoélectriques, ou une combinaison de celles-ci. À cette fin, on peut citer les dispersions de silice sous forme de nanoparticules, du noir de carbone, des oxydes simples comme la magnésie, ou complexes comme LiAlO₂, les nitrures et carbures métalliques, les silicates lamellaires en particulier de type micas, hectorite, montmorillonite, vermiculite, du graphite, y compris sous forme expansée; des fluoroaluminates complexes de type KAlF₄, des fibres de type polyoléfines ou polyimides, y compris ceux aromatiques, des fibres de carbone en particulier celles obtenues par pyrolyse de matières organiques des fibres de céramiques comprenant les oxydes, nitrures ou carbure ou oxynitrures ou oxycarbures, optionnellement sous forme de nappe tissée ou non tissée. Des additifs conférant d'autres propriétés spécifiques au polymère peuvent également être ajoutés, par exemple des additifs ayant une conductivité ionique ou électronique comme l'alumine β ou β", le nitrure de lithium, le carbone sous toutes ses formes, les polymères conjugués, en particulier les dérivés du benzène, thiophène, pyrrole et noyaux hétéroaromatiques condensés. Des additifs de structure perovskite peuvent contribuer à augmenter la constante diélectrique en induisant des propriétés piézoélectriques pour le matériau composite résultant.

La proportion molaire de monomère à double fonctionnalité peut être choisie entre 0.1 et 100% molaire selon la densité de réticulation désirée. Les compositions préférées de l'invention comprennent entre 1 et 35% molaire de monomère à double fonctionnalité.

Des liquides ou plastifiants permettant d'augmenter la flexibilité du polymère à l'état fini ou pendant sa mise en forme peuvent également être ajoutés. De tels liquides ou plastifiants sont très nombreux et connus de la personne du métier. En général, ces matériaux sont choisis pour leur compatibilité avec la chaîne polymère et pour leur faible volatilité. On peut citer principalement les esters de polyacides organiques, tels que les phtalates, les citrates, α,ω- diacides de 3 à 12 atomes de carbone d'alkyle ou d'alkylène glycols de 2 à 18 atomes de carbone, et les esters des acides phosphoriques ou phosphoniques qui confèrent des propriétés ignifuges.

L'ajout de plastifiants permet également d'augmenter si nécessaire la conductivité à basse température des électrolytes polymères. Le plastifiant est alors choisi en fonction de sa constante diélectrique ainsi que pour sa stabilité électrochimique dans les conditions où l'électrolyte polymère est destiné à être utilisé. Les plastifiants les plus utiles pour cet usage comprennent les carbonates cycliques et acycliques, en particulier le carbonate d'éthylène et le carbonate de propylène, le carbonate de diméthyle, de diéthyle, d'éthyle-méthyle et de méthyle-propyle; la γ-butyrolactone; les esters d'acides carboxyliques tels que les formiates, acétates, propionates d'alkyles de 1 à 6 atomes de carbone; les tétraalkylsulfamides; les éthers dialkylés des mono, di, tri et tétraéthylène glycols comprenant des groupements alkyles de 1 à 8 atomes de carbone; les éthers dialkylés des oligooxyéthylène de masses inférieures à 2000 g/mol, ces groupements alkyles pouvant avoir de 1 à 18 atomes de carbone. Ces plastifiants peuvent être utilisés seuls ou en mélanges.

La teneur en plastifiant peut être variable, généralement entre 0.5 et 90% en poids, préférablement entre 3 et 70% en poids. De hautes concentrations de plastifiants conduisent à des matériaux de conductivité élevée. La mobilité des chaînes devenant importante, ceci se traduit en contrepartie par une perte notable des propriétés mécaniques. La forte densité de réticulation obtenue avec les polymères selon l'invention est un avantage pour garder de bonnes propriétés mécaniques.

Les plastifiants ajoutés peuvent aussi être réactifs dans la réaction de réticulation cationique. Le ou les plastifiants additionnés au polymère doivent dans ce cas contenir au moins une fonction réactive en polymérisation cationique. De nombreux plastifiants de ce type sont connus et/ou disponibles commercialement, en particulier ceux porteurs de fonctions éther vinylique. On peut citer les éthers vinyliques des glycols, en particulier du butanediol, du di-, tri-, et tétraéthylène glycol et de leur monoalkyl-éthers, du triméthylolpropane. Un additif particulièrement intéressant de par sa haute constante diélectrique et sa facilité à dissoudre les composés polaires, sels métalliques ou d'onium, y compris les photo-amorceurs cationiques, est le propényl-propylène carbonate éther (PEPC)

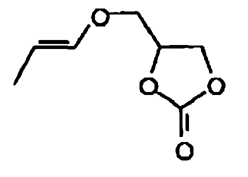

Tout autre colorant, additif anti-oxydant ou anti-UV connus de la personne du métier et compatible avec les structures des monomères et polymères en résultant peuvent être utilisés pour les polymères de l'invention.

La polymérisation des groupements Y peut être faite en présence d'autres monomères, oligomères ou polymères portant des fonctions qui peuvent être activées par les agents amorçant les polymérisations cationique, radicalaire, ou les deux. La polymérisation des groupements Y et de tout additif optionnel sous forme de monomère, oligomère ou polymère, peut être faite par voie thermique, par action de rayonnement actinique, ou les deux, en présence ou non d'un initiateur. Par exemple, la réticulation peut aussi être effectuée en présence de cycles réactifs en polymérisation cationique, tels que les époxydes, le 1,3-dioxolane, 1,3-dioxane, 1,3-dioxépane et de leurs dérivés, les spiranes de type orthoformate ou orthocarbonate définis précédemment, par exemple les dérivés du pentaérythritol mono- et di-formal. Dans le cas où Y est un éther vinylique, il est de plus possible d'incorporer des monomères possédant des doubles liaisons pauvres en électrons. Les plus représentatifs sont les fumarates, les maléates, l'anhydride maléique, les maléimides, et dans une moindre mesure, les acrylates et méthacrylates. Ces composés forment avec les éthers vinyliques des complexes de transfert de charges dont la polymérisation concertée amène la formation d'un polymère alterné formant le sous-réseau de réticulation. Ce type de polymérisation est spontanément activé par la chaleur ou les sources de radicaux libres, générés thermiquement ou par l'intermédiaire d'une radiation actinique, même en présence d'un photoamorceur. Les dérivés de la maléimide en particulier donnent des complexes spontanément polymérisables en présence de rayonnement UV et peu sensibles à l'oxygène. Les composés peuvent être monofonctionnels, ou bifonctionnels comme par exemple : 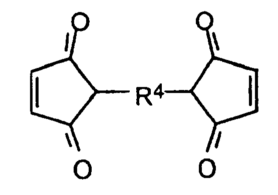
dans lequel R⁴ est un radical alkylène comprenant de 2 à 18 atomes de carbone ou oxyalkylène -CH₂CH₂(OCH₂CH₂)ₙOCH₂CH₂- dans lequel n varie entre 0 et 60.

L'utilisation des composés de l'invention pour la préparation d'électrolytes polymères représente une mise en oeuvre particulièrement préférentielle. De tels électrolytes polymères ont des applications nombreuses dans le domaine de l'électrochimie. Ils ont aussi des propriétés antistatiques ne nécessitant pas l'apport de poudres conductrices absorbantes. Comme il a été mentionné précédemment, les polyéthers sont des matériaux de choix à cause de leur pouvoir solubilisant et dissociant des sels métalliques, ou de bases azotés protonées (ammonium, imidazolium, guanidinium etc). L'architecture du polymère peut être linéaire, en étoile, ou de type peigne à chaîne latérales. Les polymères linéaires sont plus aisément obtenus par copolymérisation d'un ou plusieurs époxydes solvatants avec un monomère de l'invention possédant pour groupement A une fonction époxyde. Les époxydes solvatants comprennent préférentiellement l'oxyde d'éthylène, l'oxyde de propylène et l'oxyde de butylène. L'oxyde d'éthylène est particulièrement préféré du fait de son haut pouvoir complexant et de sa forte réactivité en polymérisation anionique, permettant ainsi un contrôle des masses moléculaires.

Il peut être souhaitable, selon les conditions d'utilisation de l'électrolyte polymère, de pouvoir minimiser le taux de cristallinité résultant de la tendance des segments polyoxyde d'éthylène à former des domaines organisés dont l'existence est préjudiciables à la conductivité. Pour cela, on choisit la préparation de terpolymères entre l'oxyde d'éthylène, le monomère de départ et un terpolymère. Le terpolymère comprend préférentiellement l'oxyde de propylène, l'oxyde de butylène, le méthylglycidyléther, l'allylglycidyléther, ou un monomère portant une fonction ionique tel que 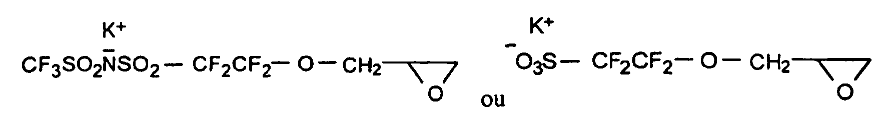 dans une proportion préférentielle de 0.5 à 25% molaire. Le potassium est avantageux parce qu'il n'interfère pas avec la polymérisation cationique et peut être échangé ultérieurement par d'autres ions, dont le cation lithium.

Le monomère des électrolytes polymères selon l'invention comprend préférentiellement les composés 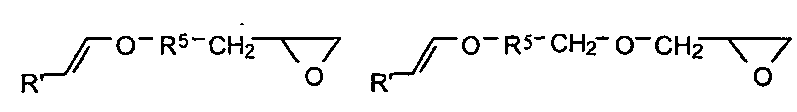 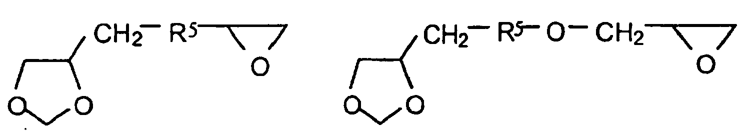 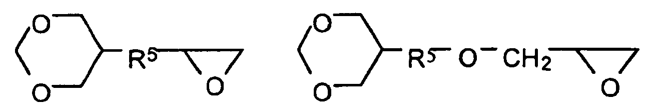 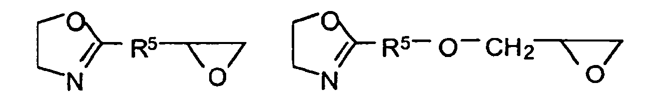 dans lesquels
R⁵ représente un radical alkyle ou oxa-alkyle divalent de 0 à 12 atomes de carbone; et
R' est tel que défini précédemment.
R⁵ est préférentiellement -CH₂-, -C₂H₄-, -C₄H₈-, -C₆H₁₂-, -C₂H₄OC₂H₄- ou -C₂H₄OC₂H₄OC₂H₄O-, et R' comprend préférentiellement H, méthyle ou éthyle. Lorsque R⁵ est -CH₂-, R' est de préférence CH₃- ou C₂H₅-. Les monomères correspondants sont aisément accessibles à partir de l'allyl glycidyl éther commercial par isomérisation de la double liaison, en présence du complexe RuCl₂ avec les phosphines ou des dérivés du fer pentacarbonyle Fe(CO₅).

Dans un autre mode de réalisation des électrolytes polymères, la polymérisation anionique du styrène est utilisée pour former une chaîne polymère à chaîne oxyde d'éthylène latérale. La formule générale de ces composés est : 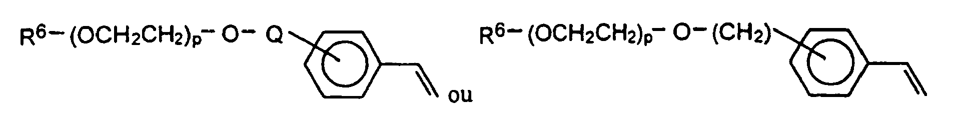
dans laquelle
p varie entre 2 et 60;
R⁶ est un radical alkyle monovalent comprenant de 1 à 18 atomes de carbone, ou aryle monovalent comprenant de 5 à 18 atomes de carbone;
Q est (CH₂)_{q}, -CO- ou -SO₂-; et
q varie entre 0 et 4.

Un exemple préféré de type de composé est lorsque Q est (CH₂)_{q} et q est 1, qui est aisément préparé par réaction d'un dérivé d'un métal alcalin d'un alcoxy oligooxyéthylène R⁶(OCH₂CH₂)ₚOM où M est Li, Na, K, sur l'ortho- ou para-chlorométhylstyrène.

Les monomères permettant de préparer les polymères préférentiels de l'invention comprennent 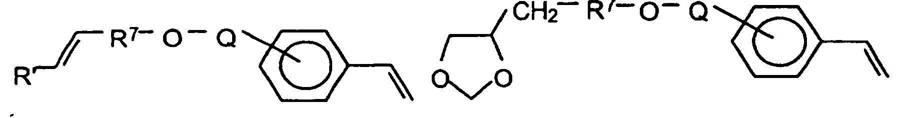 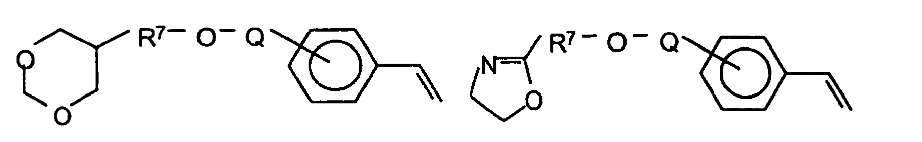
dans lesquels R' et Q sont tels que définis précédemment, et R⁷ est R⁵.

Les composés particulièrement préférés sont ceux où Q est (-CH₂-)_{q}, et q est 1.

Il peut être souhaitable d'incorporer un terpolymère de type styrènique pour introduire des variations dans le T_{g}, l'adhésion, la polarité etc. Les dérivés du vinylbenzène ayant des fonctionnalités variées représentent un bon choix, et ces derniers sont nombreux et connus de la personne du métier. Parmi les fonctionnalités d'intérêt, on note celles contenant des groupements polaires destinés à changer la constante diélectrique locale, par exemple NO₂, RCO et RCOO; les tensions de surface, par exemple les chaînes alkyles de plus de 8 carbones. Les halogènes par ailleurs agissent sur l'inflammabilité. Il est intéressant d'incorporer des monomères possédant des fonctions ioniques pour induire une conductivité ionique, principalement due aux cations qui sont les plus intéressants pour les applications électrochimiques. Dans ce cas, on peut citer les sels monomères:

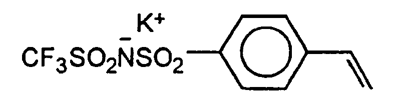

Dans un autre mode de réalisation préférentiel des électrolytes polymères, la polymérisation anionique d'une double liaison activée par un groupement carbonyle est utilisée pour former une chaîne polymère à chaîne oxyde d'éthylène latérale. La formule générale du monomère principal permettant de réaliser ces polymères est: 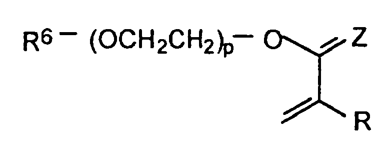
dans laquelle
R' et R⁶ sont tels que définis précédemment.

Les monomères permettant de préparer les polymères préférentiels selon l'invention comprennent: 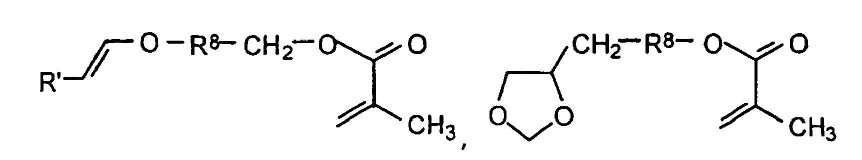 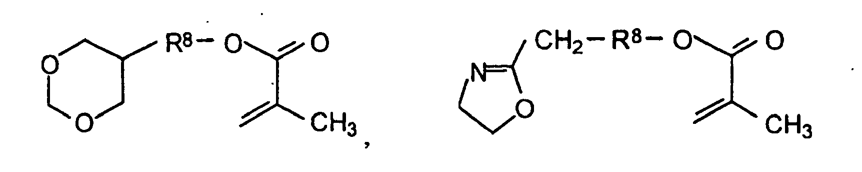
dans lesquels R' est tel que défini précédemment, et R⁸ est R⁵.

Comme précédemment, il est intéressant d'incorporer des monomères possédant des fonctions ioniques. Dans ce cas, on peut citer les sels monomères:

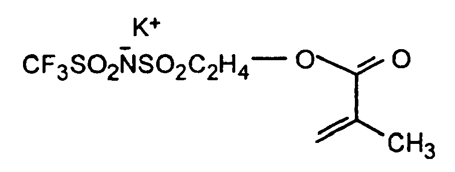

Dans les modes de préparation d'électrolytes polymères décrits ci-haut, la conductivité est assurée par un sel dissous dans la chaîne solvatant les segments polyéther. D'une manière générale, les sels sont choisis parmi les sels métalliques ou les sels d'une base azotée protonée, par exemple ammonium, imidazolium ou guanidinium, qui sont susceptibles de libérer le cation M²⁺ et un anion choisi de préférence parmi les anions peu basiques et non nucléophiles comme ClO₄⁻, BF₄⁻, PF₆⁻, AsF₆⁻, SbF₄⁻, R_{f}SO₃⁻, CₙF₂ₙ₊₁SO₃⁻ où n varie entre 0 et 8, (RₓSO₂NSO₂R'ₓ)⁻, (RₓSO₂C(SO₂R'ₓ)Rₓ")⁻, les anions dérivés du cyclopentadiène et de ses analogues aza portant des groupements électroattracteurs, les anions dérivés de pyrimidine-trione ou de la 1,3-dioxane-4,5-dione portant des groupements électroattracteurs en particulier de type CN ou CF₃SO₂, et les dérivés du malononitrile, dans lesquels Rₓ et Rₓ' sont des groupements identiques ou différents et dont au moins un possède des atomes électronégatifs tel qu'un halogène, en particulier tel que un au moins Rₓ et Rₓ' est égal CₙF₂ₙ₊₁ où n varie entre 0 et 8, Rₓ" étant soit Rₓ, soit RₓSO₂- ou Rₓ'SO₂⁻. Les anions préférentielles sont ClO₄⁻, BF₄⁻, PF₆⁻, CF₃SO₃⁻, (FSO₂)₂N⁻, (CF₃SO₂)₂N⁻, (C₂F₅SO₂)₂N⁻, [(CF₃SO₂)₂CH]⁻, [CF₃SO₂)C(CN)₂]⁺, [(CF₃SO₂)₃C]⁻, (CF₃SO₂)NSO₂N(R⁹)₂)⁻ où R⁹ représente alkyle de 1 à 30 atomes de carbone, les anions dérivés du 4,5-dicyano-1,2,3-triazole, du 3-5-bistrifluorométhyl-1,2,4-triazole, ou du tricyanométhane. La concentration de sel est plus commodément exprimée en termes de rapport du nombre d'oxygènes des segments oxyéthylènes par cation (O/M). D'une manière générale, ce nombre est compris entre 0.5 et 1000.

Un grand nombre de cations ou de mélange de cations donnent des solutions dans les polymères de l'invention. Les cations du lithium, sodium, potassium, calcium, de l'étain, ammonium et imidazolium, sont préférés. L'ion lithium et les mélanges d'ions lithium et potassium sont tout particulièrement préférés pour les applications électrochimiques.

Dans un mode de réalisation, au moins une fraction des anions est portée par la chaîne du polymère, ou bien fait partie d'une chaîne polymère différente, en mélange ou formant un réseau interpénétré avec le polymère.

La partie anionique ou la partie cationique du sel peuvent faire partie d'une chaîne macromoléculaire, y compris du polymère de l'invention, par l'incorporation de termonomères précités ou autres de type ionique. Les polymères dans lesquels une partie au moins des charges négatives sont fixées sur le polymère sont particulièrement utiles en tant qu'électrolytes solides pour les applications dans les batteries et accumulateurs, les supereapacités ou les systèmes électrochromes.

Comme il a été mentionné précédemment, les polymères selon l'invention peuvent être réticulés jusqu'à des hauts taux de réticulation du fait de la réactivité des fonctions cationiques des monomères employés. Dans le cas des électrolytes polymères, des taux de réticulation élevés destinés à induire de bonnes propriétés mécaniques sont utiles si les températures de transition vitreuse ne sont pas augmentées d'une manière notable. Ainsi qu'il a été exposé, la flexibilité des liens reliant la chaîne anionique au sous-réseau cationique ainsi que la flexibilité intrinsèque de ce sous-réseau sont des atouts déterminants pour les électrolytes polymères.

La mise en forme des électrolytes polymères en films minces est faite de façon conventionnelle par épandage à partir de solutions ou par extrusion. Pour des raisons économiques et minimisation d'impact sur l'environnement, il est souhaitable d'utiliser les plus petites quantités possibles de solvants. À ce point de vue, les polymères de faibles masses sont intéressants car leur mise en forme peut se faire à partir de solutions concentrées ou à l'état pur. Dans le cas des époxydes, les extrémités de chaînes sont en général des fonctions hydroxyles. Leur concentration plus élevée dans le cas de polymères de faibles masses est préjudiciables dans la mesure où ces fonctions sont réactives, en particulier vis-à-vis du lithium. Un avantage de la réticulation cationique est que les extrémités de chaînes obtenues après polymérisation anionique peuvent réagir au cours de l'activation des groupements Y pour obtenir la réticulation ou co-réticulation pour donner des liens stables de type acétal, par exemple la neutralisation de fonctions hydroxyles forme des liens acétals stables aux potentiels proches de ceux du lithium participant à la réticulation.- Cette possibilité est exemplifiée ci-dessous dans le cas où les fonctions cationiques sont des éthers vinyliques :

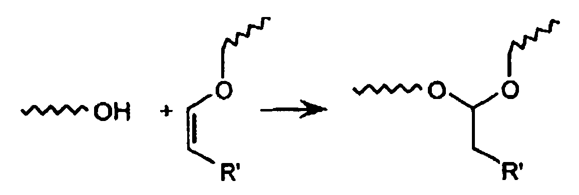

Un autre objet de l'invention est une cellule électrochimique dont l'électrolyte est constitué par un matériau à conduction ionique comprenant un polymère selon la présente invention.

Les polymères de la présente invention peuvent être utilisés en combinaison avec au moins un sel Mⁿ⁺Xₙ⁻, Mⁿ⁺ étant un cation inorganique, organique ou organométallique quelconque de charge n et X⁻ étant un anion monovalent, deux ou plusieurs anions X⁻ pouvant être liés ensemble par des chaînes covalentes ou pouvant appartenir à la chaîne d'un polymère. Une telle combinaison est utile dans un système de stockage de l'énergie électrique de type générateur primaire ou secondaire ou super-capacité comprenant au moins une électrode négative et au moins une électrode positive, cette dernière comprenant au moins en partie le composé ionique. L'électrode positive peut en outre comprendre un autre matériau d'électrode tel que les oxydes de vanadium Li_{y}VOₓ dans lequel (2x-5 ≤ y ≤ 2x-3; 2.15 ≤ x ≤ 2.5), Li_{y}N_{1-x-z}CoₓAl_{z}O₂ où 0 ≤ x + y ≤ 1 et 0 ≤ y ≤ 1, les spinelles de manganèse Li_{y}Mn₂₋ₓMₓO₄ où M est Li, Cr, Al, V, Ni, 0 ≤ x ≤ 0.5 et 0 ≤ y ≤ 2, les polydisulfures organiques, les polyquinones telles les rhodizonates, FeS, FeS₂, le sulfate de fer, les phosphates et phosphosilicates de fer et de lithium de structure olivine ou Nasicon, ou les produits de substitution du fer par le manganèse, utilisés seuls ou en mélange. L'électrode négative peut comprendre le lithium métallique, un des ses alliages, optionnellement sous forme de dispersion nanométrique dans l'oxyde de lithium, les nitrures doubles de lithium et d'un métal de transition, les oxydes à bas potentiel de formule générale Li_{1+y}Ti_{2-x/4}O₄, où x ≥ 0 et y ≤ 1, MoO₂, WO₂, le carbone et les produits carbonés issus de la pyrolyse de matières organiques, les alliages lithium-aluminium ou lithium-silicium.

Dans un système de stockage d'énergie selon la présente invention, le matériau peut comprendre un plastifiant comprenant les liquides polaires choisis parmi le carbonates cycliques et acycliques, la γ-butyrolactone, les esters d'acides carboxylique, les tétraalkylsulfamides, les éthers dialkylés des mono, di, tri et tétraéthylene glycols, les oligomères de masses inférieures à 2000 g/mol, et leurs mélanges.

Un autre objet de l'invention est un système de modulation de la lumière comprenant deux supports, dont au moins un est transparent, recouverts d'un conducteur à base d'oxyde d'étain ou de d'oxyde d'indium dopé et d'une couche de matériau électrochrome et d'une contre-électrode, dans lequel l'électrolyte est un matériau selon la présente invention. Un tel système peut comprendre, en plus du matériau, au moins un colorant susceptible de changer de coloration selon son degré d'oxydation.

Les exemples suivants sont fournis afin d'illustrer des modes de réalisations préférentiels de l'invention.

### Exemple 1

Dans un réacteur de 500 mL, 110 g d'un polymère trifonctionnel commercial préparé par polymérisation anionique de l'oxyde d'éthylène à partir du 1,1,1-tris(hydroxyméthyl)propane sont dissous dans 250 mL de THF. 33.6 g de tertiobutoxyde de potassium sont ajoutés, puis 86 g de 4-vinyloxy-1-glycidoxy-butane: 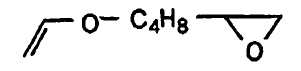 Le mélange est chauffé 3 heures à 50°C. En fin de réaction, la terminaison des chaînes est obtenue par des groupes méthyle provenant de l'addition de 37 g de sulfate de méthyle. Le polymère à bloc a pour formule : 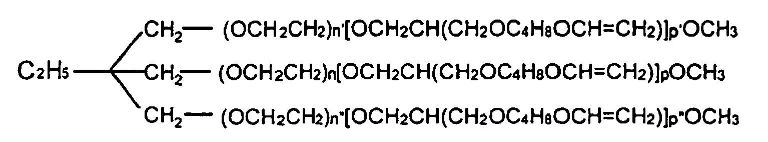
dans lequel
n + n' + n" est de l'ordre de 20; et
p + p' + p" est de l'ordre de 5.

La solution polymère est centrifugée et le THF est éliminé. La purification s'effectue par mise en solution dans 200 mL de dichlorométhane et extraction à l'eau (400 mL). Le polymère est ensuite précipité dans 2 L d'éther éthylique maintenu à -20°C.

Un électrolyte polymère est obtenu par dissolution du polymère ainsi obtenu dans un mélange d'acétone (55% en poids) en présence de perchlorate de lithium, de manière à obtenir un rapport des oxygènes des groupements oxyéthylène par ion lithium de 16:1. À cette solution, sont ajoutés 1% en poids par rapport au polymère contenu dans la solution (C₄H₉OC₆H₄IC₆H₅)⁺[FSO₂)₂N]⁻. Le polymère est épandu à partir de la solution sous forme de film de 35 µm d'épaisseur sur un support de polyéthylène haute densité et réticulé par irradiation UV à 365 nm pendant 20 secondes, correspondant à une énergie de 35 mJoules/cm². Le polymère obtenu est un élastomère résilient dont la conductivité est de 10⁻⁵ Scm⁻¹ à 25°C.

### Exemple 2

Le formal du glycérol commercial est séparé en ses deux isomères (4-hydroxyméthyl-1,3-dioxolane et 5-hydrory-1,3-dioxane selon la méthode de Hibbert et al. [*J. Am. Chem. Soc.* **50,** 3120 (1928)]. À 10.4 g de 4-hydroxyméthyl-1,3-dioxolane (isomère 1) sont ajoutés sous forte agitation 5.8 g de KOH préalablement broyés dans un mixer, 200 mg de chlorure de tétrabutylammonium agissant comme catalyseur de transfert de phase. 9.2 g d'épichlorhydrine sont ensuite ajoutés progressivement de manière à permettre de maintenir la température à une valeur inférieure à 30°C par un refroidissement externe. Le KCl formé est séparé par filtration et le (4-glycidoxyméthyl)-1,3-dioxolane obtenu est purifié par deux distillations.

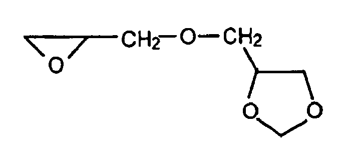

Ce monomère est copolymérisé avec l'oxyde d'éthylène dans les conditions de l'exemple 12 en utilisant le *tert*-butoxyde de potassium comme amorceur anionique. Le rapport molaire des monomères est choisi de 95 (EO) à 5 (monomère bifonctionnel). Le polymère est précipité par l'hexane. La masse moléculaire mesurée par chromatographie d'exclusion stérique est de 45 x 10³ g. Un électrolyte polymère est préparé par dissolution du sel Li(CF₃SO₂)₂N en concentration telle que O/Li = 24:1 dans un solvant commun, l'acétonitrile, auquel est ajouté 1% du sel de diméthyl-phénacyl-sulfonium [C₆H₅(=O)CH₂S(CH₃)₂]⁺(CF₃SO₂)₂N⁻. La solution est épandue à l'aide d'un gabarit pour former après évaporation du solvant un film de 24 µm d'épaisseur. La réticulation est effectué par irradiation avec une lampe UV de type Hanovia pour une dose de 40 mJ/cm². Le polymère obtenu est comme précédemment un élastomère conducteur (10⁻⁵ Scm⁻¹ à 25°C et de 1.3 x 10⁻³ Scm⁻¹ à 80°C).

### Exemple 3

Le diéthylèneglycol monovinyl éther (BASF) est estérifié par le chlorure de méthacryloyle pour former 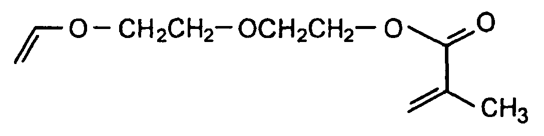 Un polymère statistique est obtenu par polymérisation anionique de ce monomère avec du méthacrylate de ω-méthoxy(oligooxyéthylène) dont la masse du segment oxyéthylène est de 900. Le rapport des deux monomères est de 85:15 (oligo-EO/dioxolane). L'amorceur anionique utilisé est la phosphazène base P4-*t*-bu dans le THF anhydre. La polymérisation est effectuée à 25°C en 24 heures. Le polymère est précipité par l'éther diéthylique, et purifié par deux dissolution/précipitation dans le couple de solvants THF/éther. Le polymère est mis sous forme de film mince par épandage à partir d'une solution dans l'acétonitrile contenant du trifluorométhanesulfonate de lithium en concentration telle que le rapport des concentrations des atomes d'oxygène de type éther apportés par le sel de lithium soit de 20:1. Un amorceur thermique cationique, le toluènesulfonate de 2-nitrobenzyle, est ajouté à raison de 0.8 % en poids. On ajoute aussi de la silice colloïdale à raison de 12% en poids du polymère. La viscosité du mélange est ajustée de manière à former par évaporation un film de 45 µm d'épaisseur. Le film ainsi obtenu est réticulé par chauffage 1 heure à 80°C. L'électrolyte polymère obtenu est un élastomère résilient dont la conductivité est de 2 x 10⁻⁵ Scm⁻¹ à 25°C.

### Exemple 4

11.6 g de 1,4-butanediol monovinyl éther (BASF) sont traités dans 100 mL de THF par 2.6 g d'hydrure de sodium. La solution obtenue est mise à réagir au reflux sous azote avec 15.2 g de chlorométhyl-4-styrène commercial (Aldrich). Le 4-vinyloxybutoxyméthyl-4-styrène 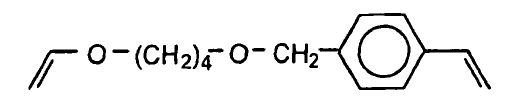 est obtenu après filtration et évaporation du THF. Le monomère est purifié par distillation. Un polymère de styrène et de 4-vinyloxybutoxyméthyl-4-styrène (91:9 molaire) est préparé par amorçage par le *sec*-butyllithium (5 x 10⁻³ molaire/total des monomères) dans le toluène à -30 °C. Après 8 heures, le polymère est précipité dans le méthanol.

Le photoamorceur cationique bis(trifluorométhanesulfonylimidure de 4-octyloxyoxyphényl(diphényl)sulfonium est préparé par échange dans l'eau entre l'hexafluorophosphate de sulfonium correspondant, commercial, et le sel de potassium de l'imidure K(CF₃SO₂)₂N. Un film est préparé par évaporation d'une solution du copolymère de polystyrène substitué et 0.5% en poids du photo amorceur dans le toluène de manière à obtenir un film de 10 mm d'épaisseur sur un substrat d'aluminium. Le polymère est soumis à un rayonnement UV à 254 nm à 40 mJ/cm²) pendant 5 secondes. Le polymère réticulé est insoluble dans tous les solvants usuels et présente une très bonne rigidité diélectrique.

### Exemple 5

5 g de polymère styrènique de l'exemple 4 et 400 mg d'anhydride maléique sont mis en solution dans 15 mL de THF et épandus sous forme de film sur un substrat d'aluminium de 8 µm d'épaisseur. Le polymère est soumis à un rayonnement UV à 254 nm à une dose de 60 mJ/cm². Le film obtenu réticulé est insoluble dans tous les solvants usuels et son adhésion au substrat d'aluminium permet de déformer celui-ci (mandrel test : 4 mm, "ASTM standard D 3359 crosshatch adhesion test: 5) sans décollement appréciable du polymère.

### Exemple 6

On fait réagir 10.4 g de 5-hydroxy-2,3-dioxane (isomère 2 de l'exemple 2) sur 9.65 ml de chlorure de méthacryoyle en présence de 8 g de pyridine à 0°C dans 50 mL de THF pour obtenir le 5-méthacryloxy-1,3-dioxane qui est purifié par distillation.

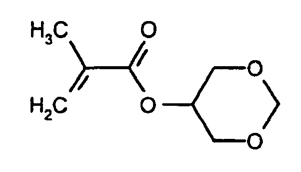

Un copolymère de méthacrylate de méthyle, de butyle et de 5-méthacryloxy-2,3-dioxane est préparé par amorçage anionique par le thiophénoxyde de tétrabutylammonium (FLUKA) dans le THF. Les rapports molaires sont 65:25:10. Le polymère est précipité dans l'éthanol et purifié par deux dissolutions / précipitations dans le couple THF:éthanol. Un film de ce polymère, additionné de 0.8 % en masse de toluènesulfonate de 2,4-dinitro-benzyle, obtenu par épandage d'une solution dans le THF sur un support de polytétrafluoroéthylène, est réticulé par chauffage 4 minutes à 80 °C. Ce polymère est transparent et insoluble dans tous les solvants usuels. À 300°C, le polymère se reconvertit en ses monomères et peut être ainsi recyclé.

### Exemple 7

La 2-hydroxyéthyloxazoline est préparée par déshydratation azéotropique du sel formé entre l'acide 3-hydroxypropionique et l'aminoéthanol. La 4-(2-méthacryloxyéthyl)-1,3-oxazoline est préparée par action du chlorure de méthacryloyle sur le dérivé hydroxylé en présence de triéthylamine à 0°C. Le monomère a pour formule 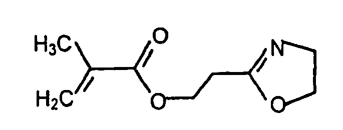 Un copolymère de méthacrylate de butyle et de 4-(2-méthacryloxy-éthyl)-1,3-oxazoline (90:10 molaire) est préparé par polymérisation anionique par la "base phosphazène P4" (Fluka) dans le THF anhydre sur 12 heures. Le polymère est précipité dans l'éthanol. Une solution à 10% en poids de ce polymère dans le dichlorométhane auquel est ajouté 0.5% en poids de busulfan® (1,4-butanediol diméthanesulfonate) est épandue sur une surface de polytétrafluoroéthylène et le solvant est évaporé. Le polymère est chauffé à 70°C pendant 10 minutes sous atmosphère d'air sec. La réticulation par ouverture cationique du cycle de l'oxazoline donne, après décollement du support, un film flexible transparent insoluble dans les solvants usuels.

### Exemple 8

13.4 g de 1,2,6-hexanetriol commercial sont additionnés de 12.4 ml de diéthoxyméthane et le mélange est porté au reflux en présence de 500 mg d'acide toluène sulfonique agissant comme catalyseur de transacétalisation. L'éthanol est éliminé à 80°C et le 4-(4-hydroxybutyl)-1,2-dioxolane obtenu est purifié par distillation. 7.3 g de ce produit et 4.6 g d'épichlorhydrine sont mis à réagir en présence de 2.8 g d'hydroxyde de potassium en poudre en présence de chlorure de tétrabutylammonium, de manière à former le monomère 4-[(4-glycidyloxy)-butyl]-1,3-dioxolane

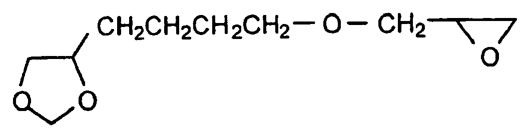

Un copolymère d'oxyde d'éthylène et de ce monomère dans le rapport molaire 93:7 est préparé par amorçage anionique par le *tert*-butoxyde de potassium dans le THF. Le polymère est purifié par centrifugation et précipitation dans l'éther. Un électrolyte polymère est formé par addition du sel de lithium Li[CF₃SO₂C(CN)₂] dans le rapport O/Li=18:1. Le polymère est réticulé par addition de 0.8% en poids de l'amorceur bisfluorosulfonyleimidure de cumène-Fe^{II}-cyclopentadiènyle [CH₃CH(CH₃)C₆H₅Fe(C₅H₅]⁺[(FSO₂)₂N]⁻ et irradation UV à la dose de 40 mJ/cm². Le polymère est un élastomère insoluble dans tous les solvants et sa conductivité est supérieure à 10⁻⁴ Scm⁻¹ à 55°C.

### Exemple 9

13.2 g de 3-allyloxy-1,2 propanediol commercial, 12 mL de triméthylorthoformate et 11.1 ml de γ-caprolactone sont chauffés à 90°C en présence de 500 mg d'acide toluènesulfonique. Le méthanol formé et l'excès d'orthoformate de méthyle sont éliminés par distillation et le spiro-ester formé est purifié par distillation. Le groupement allyle est époxydé par le sel de magnésium de l'acide monoperoxy-peroxyphtalique dans l'acétonitrile. Le monomère 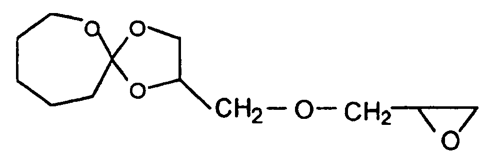 obtenu est purifié par distillation et copolymérisé avec l'oxyde d'éthylène dans les conditions de l'exemple 12 dans un rapport molaire 93:7. Le polymère obtenu est transformé en conducteur ionique par ajout du sel Li[(CH₃)₂NSO₂NSO₂CF₃] dans le rapport O/Li = 14:1. L'ajout de bis-(trifluorométhane sulfonate de 4-fluoropyridinium à raison de 1% en poids permet la réticulation du polymère à 80°C avec augmentation de volume.

### Exemple 10

15 g de ω-méthoxy(polyéthylène glycol) de masse 900 commercial sont traités dans 100 ml de THF anhydre par 600 mg d'hydrure de sodium. Après cessation du dégagement d'hydrogène, l'excès d'hydrure de sodium est éliminé par centrifugation et à la solution sont ajoutés 2.5 g de 4-chlorométhyl-styrène. Le sous-produit de la réaction (NaCl) est éliminé par centrifugation et le macromonomère est séparé par précipitation dans un mélange éther-hexane (50:50) maintenu à -20°C. Un copolymère de ce monomère et du vinyl-éther styrènique de l'exemple 4 est préparé par polymérisation anionique dans un rapport molaire 85:15 par amorçage par le *sec*-butyllithium additionné de tétraméthyléthylène diamine de manière à obtenir un polymère de masse 2.5 x 10³. Ce polymère est transformé en électrolyte polymère par dissolution de Li(CF₃SO₂)₂N dans le rapport O_{éther}/Li de 25:1. Le polymère est réticulé par addition de bis-fluorosulfonylimidure de diméthyl-phénacyl-sulfonium [C₆H₅C(=O)CH₂S(CH₃)₂]⁺(CF₃SO₂)₂N⁻ (1% en poids) et irradiation à 265 nm à la dose de 40 mJ/cm².

### Exemple 11

Un dispositif électrochrome sur support souple est élaboré par dépôt pulvérisation cathodique d'une couche de 700 nm d'oxyde de tungstène WO₃ sur un support de polyéthylène téréphtalate (PET) de 60 µm d'épaisseur recouvert préalablement d'oxyde d'étain dopé (SnO₂:F) (100 nm). La contre-électrode est un film du mélange d'oxydes Li_{0.5}TiO₂CeO₂ (800 nm) sur le même support. Les deux électrodes sont laminées de part et d'autre d'un film de l'électrolyte polymère de l'exemple 1 (30 µm). Le système ainsi assemblé, dont les bords sont scellés après ajout des amenées de courants, (bandelettes de cuivre de 0.5 cm et de 20 µm d'épaisseur) permet de faire varier la transmission de la lumière du spectre solaire de 85 à 8% par application d'un potentiel de 2V pendant 300 secondes à 25°C. L'inversion de polarité ramène la transmission à sa valeur initiale. Le système peut être cyclé sur 10⁴ cycles sans perte des propriétés optiques.

### Exemple 12

Cette exemple illustre la préparation d'un polyépoxyde réticulable selon l'invention. La polymérisation anionique a été effectuée dans un réacteur commercial en acier inoxydable Parr® d'une capacité de 2 litres et muni d'un agitateur et d'une vanne de pied permettant de le vider par le bas. Toutes les opérations de transfert ont été effectuées sous atmosphère inerte en utilisant de l'argon ou de l'azote très sec et sans oxygène. On peut également utiliser des réacteurs d'autres capacités ou dont l'intérieur est recouvert de verre.

Le réacteur utilisé a été séché de la manière suivante. On a introduit sous vide 250 ml de toluène sec, et le réacteur est ensuite chauffé à 150°C pendant 15 minutes. Le toluène est alors vidé à chaud à travers la vanne de pied. On pompe ensuite sous vide pendant environ 10 minutes.

Le monomère à double fonctionnalité 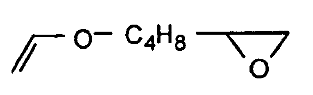 a été préparé par réaction du butanediol monovinyléther (BASF) sur l'épichlorhydrine en présence de KOH en poudre et d'un agent de transfert de phase, soit l'hydrogénosulfate de tétrabutyl ammonium. L'oxyde d'éthylène a été distillé, les solvants et les autres monomères ont été séchés sur tamis moléculaire avant leur introduction dans le réacteur, en vue d'abaisser leur teneur en eau à moins de 100 ppm, valeur vérifiée par la méthode de Karl-Fischer.

Dans le réacteur vide et exempt de traces d'eau et d'impuretés, on a introduit à la température ambiante de 20°C un mélange de 30.03 g de (4-vinyloxybutyl)-glycidyléther, 50.05 g d'oxyde de butylène et 120.2 g d'oxyde d'éthylène. On a ensuite introduit 20 x 10⁻³ mole de *tert*-butoxyde de potassium dans 20 ml de THF. La température a été maintenue à 25°C pendant 23 heures pour donner 190 g d'un mélange liquide à la température ambiante auquel on ajoute 0.2 g de Santonox R® comme antioxydant et stabilisant. Selon les proportions initiateur/monomère, la masse moléculaire est inférieure à 10,000.

### Exemple 13

Pour cet exemple, la polymérisation a été effectuée dans le réacteur selon la procédure de l'exemple 12, et les monomères ont été séchés d'une manière similaire à cet exemple.

Dans le réacteur contenant 151 g de toluène, on introduit un mélange de 11.39 g de 4-(vinyloxybutyl)-glycidyléther et 88.6 g d'oxyde d'éthylène. La température du réacteur a été portée à 100°C et on introduit alors 2 x 10⁻³ mole de *tert*-butoxyde de potassium dans 2 mL de THF. La température du réacteur est maintenue à 100°C pendant 4.5 heures. On observe une baisse de pression de 6.8 x 10⁵ Pa à 1.2 x 10⁵ Pa. On abaisse ensuite la température à 50°C et on ajoute 0.1 g de Santonox R® dissous dans 8 ml de toluène. On récupère la solution du réacteur à travers la vanne de pied. Après évaporation du solvant, on obtient 89 g de copolymère dont la masse moléculaire moyenne Mᵥ est de 39,000. La masse moléculaire moyenne Mᵥ a été évaluée par comparaison de la viscosité d'une solution du polymère avec la viscosité de solutions de poly(oxyde d'éthylène) de masse connue.

### Exemple 14

Pour cet exemple, la polymérisation a été effectuée dans un réacteur selon la procédure de l'exemple 12, et les monomères ont été séchés d'une manière similaire à cet exemple.

Dans le réacteur contenant 151 g de toluène, on introduit un mélange de 11.42 g de (4-vinyloxybutyl)-glycidyléther, 3.2 g d'oxyde de butylène et 79.8 g d'oxyde d'éthylène. La température du réacteur est portée à 100°C et on introduit 112 mg (10⁻³ mole) de *tert*-butoxyde de potassium dans 1 ml de THF. Après 1.5 heure, on ajoute 5 x 10⁻⁴ mole de *tert*-butoxyde de potassium dans 0.5 ml de THF. La température est maintenue à 100°C pendant 17 heures supplémentaires. On observe une baisse de pression de 5.9 x 10⁵ Pa à 1.5 x 10⁵ Pa. On abaisse ensuite la température à 70°C et on ajoute 0.1 g de Santonox R® dissous dans 8 mL de toluène. On récupère la solution du réacteur à travers la vanne de pied. Après évaporation du solvant, on obtient 70 g de copolymère dont la masse moléculaire moyenne Mᵥ est de 46,000, telle que mesurée par la méthode comparative de l'exemple 13.

### Exemple 15

Pour cet exemple, toutes les manipulations sont effectuées dans une boîte à gants sous atmosphère inerte et anhydre (≤ 1 vpm H₂O, O₂). Une solution A est obtenue par dissolution du polymère de l'exemple 13 dans 100 mL du mélange de solvants acétonitrile:toluène (4:1) à une concentration de 0.5 g/cc. Après dissolution complète du polymère, une solution B est obtenue par la dissolution dans la solution A de bis(trifluorométhanesulfonimidure) de lithium, LiN(SO₂CF₃)₂, de façon à obtenir un rapport oxygène du polymère sur lithium, O/Li, de 30:1. Une solution C est obtenue par la dissolution dans la solution B de 1% en poids par rapport au polymère de bis(pentafluoroéthanesulfonylimidure de diphényliodonium, [N(SO₂C₂F₅)₂]⁻[C₆H₅)₂I]⁺, qui est un amorceur de polymérisation cationique activé lors d'une exposition au rayons ultraviolets. La matrice polymère est mise en forme sous forme d'un film mince d'environ 30 µm sur un support pelable de polypropylène de 28 µm, par une méthode classique d'enduction par voie solvant de la solution C, puis séchage. La matrice polymère obtenue est subséquemment réticulée par une irradiation UV centrée sur la longueur d'onde 365 nm pendant 10 secondes à une puissance de 3300 µW/cm². L'électrolyte polymère obtenu présente de bonnes propriétés mécaniques et est insoluble dans le mélange de solvant acétonitrile:toluène (4: 1). La matrice polymère est par la suite séchée sous vide à 85°C pendant deux heures. Une mesure par DSC effectuée sur la matrice surfondue, donne une température de transition vitreuse à demi-hauteur de -65°C. Cette mesure, ainsi que celle effectué pour les exemples suivants indique que la température de transition vitreuse n'est pas affectée par la réticulation. À titre de comparaison, l'homopolymère PEO a un température de transition vitreuse de -66°C.

### Exemple 16

Dans les conditions de manipulation de l'exemple 15, une solution A est obtenue par dissolution du polymère de l'exemple 13 dans 100 ml du mélange de solvant acétonitrile:toluène (4 :1) à une concentration de 0.5 g/cc. Après dissolution complète du polymère, une solution B est obtenue par la dissolution dans la solution A de 1% en poids par rapport au polymère de [N(SO₂C₂F₅)₂]⁻[C₆H₅)₂I]⁺. La matrice polymère est mise en forme sous forme d'un film mince d'environ 30 µm sur un support pelable de polypropylène de 28 µm, par une méthode classique d'enduction par voie solvant de la solution B. La matrice polymère obtenue est réticulée par une irradiation UV centrée sur la longueur d'onde 365 nm pendant 10 secondes à une puissance de 3300 µW/cm². La matrice que l'on obtient présente de bonnes propriétés mécaniques et est insoluble dans les solvants du polymère initial. La matrice polymère est par la suite séché sous vide à 85°C pendant deux heures. Une mesure par DSC effectuée sur la matrice surfondue, donne une température de transition vitreuse à demi-hauteur de -68°C.

### Exemple 17

Dans les conditions de manipulation de l'exemple 15, une solution A est obtenue par dissolution du polymère de l'exemple 14 dans 100 ml du mélange de solvant acétonitrile:toluène (4:1) à une concentration de 0.5 g/cc. Après dissolution complète du polymère, une solution B est obtenue par la dissolution dans la solution A de bis(trifluorométhanesulfonimidure) de lithium, LiN(SO₂CF₃)₂, de façon à obtenir un rapport O/Li de 30:1. Une solution C est obtenue par la dissolution dans la solution B de 1% en poids par rapport au polymère de [N(SO₂C₂F₅)₂]⁻[C₆H₅)₂I]⁺. La matrice polymère est épandue sous forme d'un film de 30 µm sur le support de polypropylène. La matrice polymère obtenue est réticulée dans les conditions mentionnées précédemment. La matrice obtenue présente de bonnes propriétés mécaniques et est insoluble dans le mélange de solvant acétonitrile:toluène (4:1). La matrice polymère est par la suite séchée sous vide à 85°C pendant deux heures. Une mesure par DSC indique une température de transition vitreuse de -64°C.

### Exemple 18

Dans les conditions de manipulation de l'exemple 15, une solution A est obtenue par dissolution du polymère de l'exemple 14 dans 100 ml du mélange acétonitrile:toluène (4:1) à une concentration de 0.5 g/mL. Après dissolution complète du polymère, une solution B est obtenue par la dissolution dans la solution A de 1% en poids par rapport au polymère de [N(SO₂C₂F₅)₂]⁻[C₆H₅)₂I]⁺. La matrice polymère est épandue sous forme d'un film mince 30 µm sur un support pelable de polypropylène tel que décrit précédemment. Après réticulation et séchage dans les conditions de l'exemple 14, la matrice polymère obtenue a une température de transition vitreuse mesurée par DSC, de -66° C.

### Exemple 19

Dans les conditions de manipulation de l'exemple 15, une solution est obtenue par dissolution dans le polymère de l'exemple 12 de bis(trifluorométhanesulfonimidure) de lithium, LiN(SO₂CF₃)₂, de façon à obtenir un rapport O/Li de 30:1. À cette solution est ajouté 1% en poids par rapport au polymère de [N(SO₂C₂F₅)₂]⁻[C₆H₅)₂I]⁺. La matrice polymère épandue sous forme d'un film mince selon la procédure de l'exemple 15. Le polymère est réticulé par 10 secondes d'irradiation UV à 365 nm à une puissance de 3300 µW/cm². La matrice que l'on obtient présente de bonnes propriétés mécaniques et est insoluble dans les solvants du polymère avant réticulation. Une mesure par DSC indique une température de transition vitreuse de -65°C.

### Exemple 20

Cet exemple vise à illustrer l'utilité des polymères de l'invention pour préparer des électrolytes plastifiés. Toutes les manipulations sont effectuées dans une boîte à gants sous atmosphère inerte et anhydre (≤ 1 vpm H₂O, O₂). Le polymère de l'exemple 1 est dissous dans un mélange de γ-butyrolactone avec du carbonate d'éthylène (50:50 v:v) contenant de l'hexafluorophosphate de lithium à une concentration de 1 mole par litre. À ce mélange est ajoutée 1% en poids par rapport au polymère de [N(SO₂C₂F₅)₂]⁻[C₆H₅)₂I]⁺. Le mélange est épandu sous forme d'un film mince d'environ 30 µm sur un support pelable de polypropylène de 28 µm et n'est pas séchée. La matrice polymère plastifiée obtenue est réticulée par 10 secondes d'irradiation dans les conditions de l'exemple 15, pour donner un élastomère présentant de bonnes propriétés mécaniques malgré sa teneur élevée en plastifiants liquides qui ne sont pas exsudés. La conductivité est supérieure à 10⁻³ Scm⁻¹ à 25°C.

### Exemple 21

Dans les conditions de manipulation de l'exemple 15, une solution A est obtenue par dissolution du polymère de l'exemple 13 dans 100 mL du mélange acétonitrile:toluène (4:1) à une concentration de 0.5 g/cc. Après dissolution complète du polymère, une solution B est obtenue par la dissolution dans la solution A du sel LiN(SO₂CF₃)₂ de façon à obtenir un rapport O/Li de 30:1. Une solution C est obtenue par dissolution du polyoxyéthylène de masse molaire 200 000 dans 100 mL du mélange acétonitrile:toluène (4:1) à une concentration de 0.5 g/cc. Après dissolution complète du polyoxyéthylène, une solution D est obtenue par la dissolution dans la solution C de LiN(SO₂CF₃)₂ de façon à obtenir un rapport O/Li de 30:1. Une solution E est obtenue par le mélange d'une portion de chacune des solutions B et D. La proportion de solution B et D est ajustée de façon à obtenir dans la solution E une proportion volumique de polymère de la solution B de 80% et du polymère de la solution D de 20%. Une solution F est obtenue par la dissolution dans la solution E de 1% en poids par rapport aux polymères de [N(SO₂C₂F₅)₂]⁻[C₆H₅)₂I]⁺. La solution F est épandue selon la technique décrite précédemment. Le polymère obtenu est réticulé selon la procédure de l'exemple 15. La matrice obtenue présente de bonnes propriétés mécaniques, et sa température de transition vitreuse est de -62°C.

### Exemple 22

Cet exemple illustre l'ajout d'un plastifiant réactif co-polymérisable. Dans les conditions de manipulation de l'exemple 15, une solution A est obtenue par dissolution du polymère de l'exemple 13 dans 100 ml du mélange acétonitrile:toluène (4:1) à une concentration de 0.5 g/cc. Après dissolution complète du polymère, une solution B est obtenue par dissolution dans la solution A de LiN(SO₂CF)₂ de façon à obtenir un rapport O/Li de 30: 1. Une solution C est obtenue par dissolution dans du triéthylèneglycol divinyléther (DVE-3, ISP) de LiN(SO₂CF₃)₂ de façon à obtenir un rapport O/Li de 30:1. Une solution D est obtenue par le mélange d'une portion de chacune des solutions B et C. La proportion de solution B et C est ajustée pour obtenir dans la solution D une proportion volumique de polymère de la solution B de 80% et du polymère de la solution C de 20%. Une solution E est obtenue par la dissolution dans la solution D de 1.2% en poids par rapport aux polymères de [N(SO₂C₂F₅)₂]⁻[C₆H₅)₂I]⁺. La solution E est épandue sous forme d'un film mince selon la procédure mentionnée précédemment. La matrice polymère obtenue est réticulée suite à 10 secondes d'irradiation UV à 365 nm à une puissance de 3300 µW/cm². Le film présente de bonnes propriétés mécaniques et est insoluble dans les solvants du polymère. Un polymère similaire est obtenu par remplacement du DVE-3 de la solution C par le triméthylolpropane trivinyléther. Le mélange des solutions B et C est fait dans les proportions 70:30%.

### Exemple 23

Dans les conditions de manipulation de l'exemple 15, on fabrique un générateur électrochimique en utilisant une électrode négative de lithium métallique de 30 µm d'épaisseur laminé sur un collecteur de courant de nickel de 8 µm. Le séparateur est constitué de la matrice polymère de l'exemple 19. L'électrode positive contient un mélange de poudre d'oxyde de vanadium, de noir de carbone (Shawinigan black®) et d'un terpolymère à base de polyéther contenant du bis(trifluorométhanesulfonimidure) de lithium dans un rapport molaire O/Li de 30:1, ladite électrode positive ayant une capacité de 6 Coulomb/cm². Le matériau composite est épandu à partir de la solution sur un collecteur de courant en aluminium de 8 µm d'épaisseur pour donner un film de 45 µm d'épaisseur. Le générateur électrochimique est assemblé par pressage à chaud à 80°C sous vide. Après 12 cycles à 80°C, le générateur présente un très bon comportement en cyclage sur le plan de l'efficacité coulombique et une capacité qui demeure constante.

### Exemple 24

Dans les conditions de manipulation de l'exemple 15, une solution A est obtenue par dissolution dans le polymère de l'exemple 1, d'hexafluorophosphate de lithium de façon à obtenir un rapport O/Li de 30:1. Une solution B est obtenue par l'ajout de 1% en poids par rapport au polymère de [N(SO₂C₂F₅)₂]⁻[C₆H₅)₂I]⁺ dans la solution A. La matrice polymère est mise en forme sous forme d'un film mince d'environ 30 µm sur un support pelable de polypropylène de 28µm, par enduction de la solution B. La matrice polymère obtenue est ensuite réticulée par 10 secondes d'irradiation UV à 365 nm à une puissance de 3300 µW/cm².

On fabrique un générateur électrochimique en utilisant une électrode négative qui contient du graphite dans une fraction massique de 90 % et un polymère de fluorure de vinylidène-*co*-hexafluoro propène, dans une fraction massique de 10%. La dite électrode négative possède une capacité de 3.56 C/cm². L'électrode est obtenue par enduction en phase solvant (acétone) sur un collecteur de courant en cuivre de 16 µm d'épaisseur pour donner un film de 56 µm d'épaisseur. Le séparateur comprend une membrane en polymère telle que décrite au paragraphe précédent (membrane polymère d'une épaisseur de 15 µm contenant de l'hexafluorophosphate de lithium dans un rapport molaire O/Li 30: 1). L'électrode positive contient un mélange de cobaltite de lithium, LiCoO₂, dans une fraction massique de 91.6%, du noir de carbone dans une fraction massique de 2.7% et un polymère de fluorure de vinylidène co-hexafluoro propène, dans une fraction massique de 5.7 %. Ladite électrode positive possède une capacité de 4.08 C/cm². L'électrode est obtenue par enduction en phase solvant (acétone) sur un collecteur de courant en aluminium de 8 µm d'épaisseur de façon à donner un film de 49 µm d'épaisseur. Au moment de l'assemblage du générateur électrochimique, le séparateur est immergé 30 minutes dans le mélange de solvant éthyl-méthyl carbonate/carbonate d'éthylène (équivolumique 1:1) contenant de l'hexafluorophosphate de lithium à une concentration de 1 molaire, puis la cathode et l'anode sont immergées 10 minutes dans la solution de LiPF₆ dans le mélange de solvant éthyl-méthyl carbonate/carbonate d'éthylène (équivolumique 1:1) contenant de l'hexafluorophosphate de lithium à une concentration de 1 molaire, puis la cathode et l'anode sont immergés 10 minutes dans la même solution. Suite à l'immersion, le solvant occupe 41 % du volume du séparateur, 51 % du volume de la cathode et 45 % du volume de l'anode. Le générateur électrochimique est par la suite rapidement assemblé par pressage léger à 25°C de l'électrode négative, du séparateur et de l'électrode positive, et mis dans un sachet étanche. Après 12 cycles à 25°C, le générateur présente un très bon comportement en cyclage sur le plan de l'efficacité coulombique et une capacité qui demeure constante.

### Exemple 25

Dans les conditions de manipulation de l'exemple 15, une solution A est obtenue par dissolution du polymère de l'exemple 12 dans un mélange de γ-butyrolactone avec du carbonate d'éthylène (50:50 v/v) contenant de l'hexafluorophosphate de lithium à une concentration de 1 molaire de façon à obtenir un rapport volumique de 50% polymère et 50% mélange de solvant. Une solution B est obtenue par ajout à A de 1 % en poids par rapport au polymère de [N(SO₂C₂F₅)₂]⁻[C₆H₅)₂I]⁺.

On fabrique un générateur électrochimique en utilisant une électrode négative qui contient du graphite dans une fraction massique de 58%, un polymère de fluorure de vinylidène co-hexafluoro propène dans une fraction massique de 5.8%, le polymère de l'exemple 12 dans une fraction massique de 3.2% et un mélange de γ-butyrolactone, de carbonate d'éthylène (50:50 v/v) et contenant le sel LiPF₆ (1 molaire), à une fraction massique de 33%. On ajoute aussi 1% en poids par rapport au polymère de l'exemple 12 de bis-fluorosulfonimide de pyridinium [N(SO₂F)₂]⁻[C₅H₅)NH]⁺, un amorceur de polymérisation cationique qui s'active de façon thermique. L'électrode est obtenue par enduction en phase solvant (2-butanone) sur un collecteur en cuivre de 16 µm d'épaisseur suivie d'un recuit de trois heures à 80°C de façon à réticuler dans l'électrode le polymère de l'exemple 12. Ladite électrode négative possède une capacité de 3.6 C/cm². L'électrode positive contient un mélange de phosphate double de fer et de lithium (LiFePO₄) dans une fraction massique de 58%, un polymère de fluorure de vinylidène co-hexafluoro propène dans une fraction massique de 5.8%, le polymère de l'exemple 12 dans une fraction massique de 3.2% et un mélange de γ-butyrolactone/carbonate d'éthylène (50:50) contenant l'hexafluorophosphate de lithium 1 molaire, dans une fraction massique de 33%. On ajoute aussi 1% en poids par rapport au polymère de l'exemple 12 de [N(SO₂F)₂]⁻[C₅H₅)NH]⁺. L'électrode est obtenue par enduction en phase solvant (butanone) sur un collecteur de courant en aluminium de 8 µm d'épaisseur suivie d'un recuit de trois heures à 80°C de façon à réticuler dans l'électrode le polymère de l'exemple 12. La dite électrode positive possède une capacité de 3.99 C/cm². La solution B est épandue par enduction directement sur l'électrode négative sous forme d'un film mince de 15 µm et réticulée par 10 secondes d'irradiation UV à 365 nm à une puissance de 3300 µW/cm². Le générateur électrochimique est par la suite rapidement assemblé par pressage léger à 25°C de la demi-pile constituée de l'électrode négative et du séparateur et de l'électrode positive et mis dans un sachet étanche. Après 8 cycles à 25°C, le générateur présente un très bon comportement en cyclage sur le plan de l'efficacité coulombique et une capacité qui demeure constante.

### Exemple 26

Pour cet exemple, la polymérisation a été effectuée dans un réacteur selon la procédure de l'exemple 12, et les monomères ont été séchés d'une manière similaire à cet exemple.

Le 1-propényl glycidyl éther a été préparé par isomérisation de l'allyl-glycidyl éther commercial à l'aide du catalyseur RuCl₂[P(C₆H₅)₂]₃ à raison de 0.5% molaire à 120°C. Dans le réacteur vide et exempt de traces d'eau et d'impuretés et contenant 500 g de toluène, on introduit à la température ambiante un mélange de 5.67 g de 1-propenyl glycidyl éther, 16.1 g d'oxyde de butylène et 177.6 g d'oxyde d'éthylène. La température du réacteur est amenée à 99°C et on introduit 2 x 10⁻³ mole de tert-amylate de potassium dans 2 ml de toluène. La température est maintenue à 99°C pendant 24 heures.

On observe une baisse de pression de 12.3 x 10⁵ Pa à 3.4 x 10⁵ Pa. On abaisse ensuite la température à 45°C et on ajoute 0.2 g de Santonox R® dissous dans 8 ml de toluène. On récupère alors la solution du réacteur à travers la vanne de pied. Après évaporation du solvant, on obtient 180 g de copolymère dont la masse moléculaire moyenne Mᵥ est de 44,000 mesurée par la méthode comparative de l'exemple 13.

### Exemple 27

Pour cet exemple, la polymérisation a été effectuée dans un réacteur selon la procédure de l'exemple 12, et les monomères ont été séchés d'une manière similaire à cet exemple.

Dans le réacteur exempt de traces d'eau et d'impuretés et à la température ambiante de 20°C, on introduit un mélange de 20.05 g de 1-propenyl glycidyl éther, 60.10 g d'oxyde de butylène et 119.9 g d'oxyde d'éthylène. On introduit 20 x 10⁻³ mole de *tert-*butanolate de potassium dans 20 mL de THF et on amène la température du réacteur à 25°C. La température du réacteur est maintenue à environ 25°C pendant 26 heures. On observe une baisse de pression de 6.3 x 10⁵ Pa à 3.6 x 10⁵ Pa. On ajoute ensuite 0.2 g de Santonox R® dissous dans 8 ml de toluène. On obtient 180 g de mélange liquide visqueux du réacteur à travers la vanne de pied. À partir des proportions de monomère et de *tert*-butanolate de potassium, la masse moléculaire estimée est de l'ordre de 10 000.

### Exemple 28

Dans les conditions de manipulations de l'exemple 15, une solution A est obtenue par dissolution du polymère de l'exemple 26 dans 100 mL du mélange acétonitrile:toluène (4:1) à une concentration de 0.5 g/cc. Après dissolution complète du polymère, une solution B est obtenue par la dissolution dans la solution A de LiN(SO₂CF₃)₂, de façon à obtenir un rapport O/Li de 30:1. Une solution C est obtenue par la dissolution dans la solution B de 1% en poids par rapport au polymère de [N(SO₂C₂F₅)₂]⁻[C₆H₅)₂I]⁺. La matrice polymère est mise en forme sous forme d'un film mince selon la procédure mentionnée précédemment. La matrice polymère obtenue est réticulé suite par 12 secondes d'irradiation UV à 365 nm à une puissance de 3200 µW/cm². La matrice obtenue présente de bonnes propriétés mécaniques et est insoluble dans les solvants du polymère avant irradiation. La matrice polymère est par la suite séchée sous vide à 85°C pendant deux heures. La température de transition vitreuse est trouvée par DSC à -62°C.

### Exemple 29

Dans les conditions de manipulation de l'exemple 15, une solution A est obtenue par dissolution dans le polymère de l'exemple 20 du sel de lithium LiN(SO₂CF₃)₂ de façon à obtenir un rapport O/Li de 30:1. Une solution B est obtenue par la dissolution de 1% en poids par rapport au polymère de [N(SO₂C₂F₅)₂]⁻[C₆H₅)₂I]⁺. La solution B est épandue pour former un film de mince selon la procédure mentionnée précédemment, et réticulée par 12 secondes d'irradiation UV à 365 nm à une puissance de 3200 µW/cm². La matrice obtenue présente de bonnes propriétés mécaniques et est insoluble dans les solvants du polymère avant irradiation. La température de transition vitreuse est observée par DSC à -64°C.

### Exemple 30

Dans les conditions de manipulation de l'exemple 15, une solution A est obtenue par dissolution du polymère de l'exemple 27 dans un mélange de tétraéthylsulfamide avec de l'éthylène carbonate (60:40) contenant de l'hexafluorophosphate de lithium à une concentration de 1 molaire de façon à obtenir un rapport volumique de 50% polymère et 50% mélange de solvant. Une solution B est obtenue par la dissolution de 1% en poids par rapport au polymère de [N(SO₂C₂F₅)₂]⁻ [C₆H₅)₂I]⁺. La solution B est épandue pour former un film mince d'environ 30 µm sur un support de polypropylène, sans évaporer les solvants. La matrice polymère obtenue est réticulée par 12 secondes d'irradiation UV à 365 nm à une puissance de 3200 µW/cm². La matrice obtenue présente de bonne propriétés mécaniques et n'exsude aucun solvant.

## Revendications

1. Matériau à conduction ionique constitué par au moins un polymère et un au moins composé ionique, **caractérisé en ce que** :
- le polymère est un polymère réticulable préparé par polymérisation anionique d'un monomère répondant à la formule générale (A)ₙQ(Y)ₚ éventuellement avec un ou plusieurs autres monomères, dans laquelle:
* Q représente une liaison ou un radical organique de valence n + p non réactif vis-à-vis des agents initiant la polymérisation anionique ou cationique comprenant un radical alkyle, alkylaryle, arylalkyle comprenant optionnellement des substituants oxa ou aza, et comprenant de 1 à 30 atomes de carbone,
* A représente un radical réactif en polymérisation anionique,
* Y est un radical réactif en polymérisation cationique et non réactif vis-à-vis d'un agent initiant la polymérisation anionique et représente la fonction réticulable dudit polymère réticulable,
* n varie entre 1 et 3;
* p varie entre 1 et 6, et
- le composé ionique est constitué par au moins un sel Mⁿ⁺Xₙ⁻, dans lequel Mⁿ⁺ représente un cation inorganique, organique ou organométallique quelconque de charge n, et X⁻ est un anion monovalent, deux ou plusieurs anions X⁻ pouvant être liés ensemble par des chaînes covalentes ou pouvant appartenir à la chaîne d'un polymère.

2. Matériau selon la revendication 1, dans lequel :
A comprend au moins un des groupes suivants : 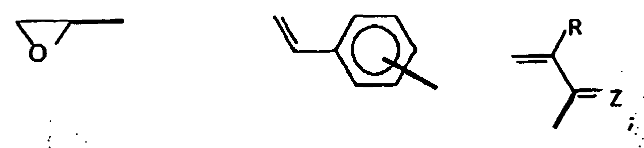 et
Y comprend au moins un des groupes suivants 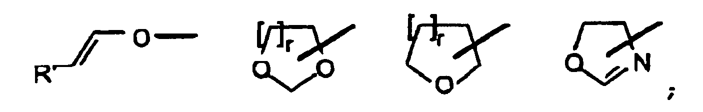
dans lesquels :
- Z représente O ou CH₂;
- R représente H, un radical alkyle ou oxa-alkyle de 1 à 12 atomes de carbone, CN ou CH₂COOR¹ dans lequel R¹ est H
ou un radical alkyle ou oxa-alkyle de 1 à 12 atomes de carbone ;
- R' représente H ou un radical alkyle de 1 à 12 atomes de carbone ; et
- r varie entre 1 et 6.

3. Matériau selon la revendication **1**, **caractérisé en ce que** le polymère est un copolymère statistique, un copolymère bloc ou un copolymère en étoile ayant des unités dérivant d'un monomère (A)ₙQ(Y)ₚ et des unités dérivées d'un ou plusieurs autres monomères.

4. Matériau selon la revendication **1, caractérisé en ce que** la mise en forme du polymère réticulable est faite avant la réticulation.

5. Matériau selon l'une des revendications 1 à **4, caractérisé en ce que** le polymère est réticulé par la polymérisation cationique des groupements Y.

6. Matériau selon la revendication **5**, **caractérisé en ce que** la polymérisation des groupements Y est faite en présence d'autres monomères, oligomères ou polymères portant des fonctions qui peuvent être activées par les agents amorçant les polymérisations cationiques, radicalaire, ou les deux.

7. Matériau selon la revendication **6, caractérisé en ce que** le monomère est choisi parmi les dérivés oxygénés des dioxolanes de type spiroorthoformates ou spiroorthocarbonates.

8. Matériau selon la revendication **5 caractérisé en ce que** la polymérisation des groupements Y et de tout additif optionnel sous forme de monomère, oligomère ou polymère, est faite par voie thermique, par action de rayonnement actinique, ou les deux, en présence ou non d'un initiateur.

9. Matériau selon l'une des revendications **1** à **8, caractérisé en ce qu'**il contient au moins un plastifiant.

10. Matériau selon la revendication **5, caractérisé en ce qu'**on y incorpore un additif sous forme de poudres, de fibres ou les deux, optionnellement traité, pour que sa surface présente une compatibilité et une adhérence accrues vis-à-vis du polymère ou des mélanges de polymères.

11. Matériau selon la revendication **10, caractérisé en ce que** l'additif est organique ou inorganique, et possède optionnellement des propriétés de conduction ionique ou électronique intrinsèques, des propriétés optiques, une constante diélectrique élevée, des propriétés piézoélectriques, ou une combinaison de celles-ci.

12. Matériau selon la revendication **1**, **caractérisé en ce que** ledit polymère est un homopolymère d'un composé (A)ₙQ(Y)ₚ dans lequel A comprend un groupe 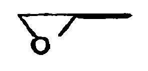
ou un copolymère d'un tel composé et d'un époxyde.

13. Matériau selon la revendication 12, caractérisé en ce l'époxyde est l'oxyde d'éthylène.

14. Matériau selon la revendication **12 caractérisé en ce que** le monomère (A)ₙQ(Y)ₚ est choisi parmi les composés : 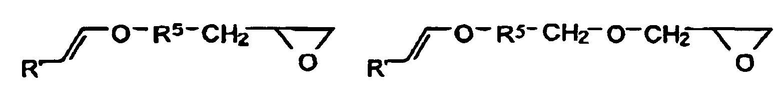 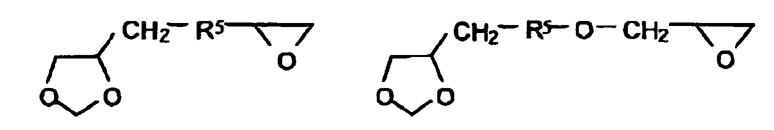 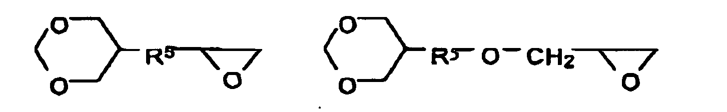 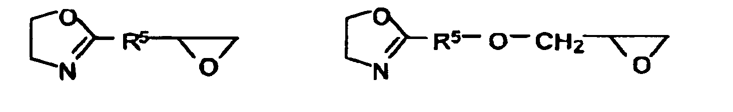
dans lesquels:
- R⁵ représente un radical alkyle ou oxa-alkyle divalent de 0 à 12 atomes de carbone; et
- R' représente H ou un radical alkyle de 1 à 12 atomes de carbone.

15. Matériau selon la revendication **13, caractérisé en ce qu'**au moins un co-monomère est utilisé en plus de l'oxyde d'éthylène.

16. Matériau selon la revendication **15, caractérisé en ce que** le co-monomère est choisi parmi l'oxyde de propylène, l'oxyde de butylène, le monoépoxyde des diènes de 4 à 10 atomes de carbone, les éthers du glycidol avec les groupements méthyle, éthyle, allyle.

17. Matériau selon la revendication 1, **caractérisé en ce que** le polymère réticulable est un homopolymère d'un composé (A)ₙQ(Y)ₚ dans lequel A comprend un groupe 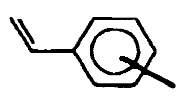
ou un copolymère d'un tel composé et d'un monomère styrénique.

18. Matériau selon la revendication **17, caractérisé en ce que** ledit monomère styrénique est le α-alkyl-(oligoéthoxy)-ω-vinylbenzyle de formule générale 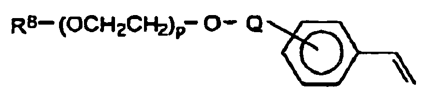
dans laquelle:
- p varie entre 2 et 60;
- R⁶ est un radical alkyle monovalent de 1 à 18 atomes de carbone, ou un radical - aryle monovalent de 5 à 18 atomes de carbone;
- Q est (CH₂)_{q}, -CO- ou -SO₂- ; et
- q varie entre 0 et 4.

19. Matériau selon la revendication **17, caractérisé en ce que** le monomère (A)ₙQ(Y)ₚ est choisi parmi 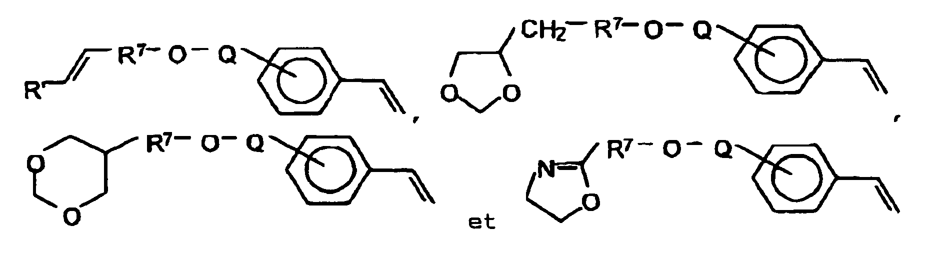
dans lesquels R' représente H ou un radical alkyle de 1 à 12 atomes de carbone, Q est (CH₂)_{q}, -CO- ou -SO₂-, et R⁷ est R⁵.

20. Matériau selon la revendication **18, caractérisé en ce qu'**au moins un autre monomère est utilisé en plus de l'α-alkyl(oligoéthoxy)-∞-vinylbenzyle.

21. Matériau selon la revendication 1, **caractérisé en ce que** le polymère réticulable est un homopolymère d'un composé (A)ₙQ(Y)ₚ dans lequel A comprend un groupe 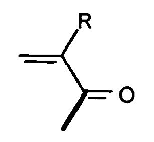
dans lequel R représente H, un radical alkyle ou oxa-alkyle de 1 à 12 atomes de carbone, CN ou CH₂COOR¹ dans lequel R¹ est H ou un radical alkyle ou oxa-alkyle de 1 à 12 atomes de carbone, ou un copolymère d'un tel composé (A)ₙQ(Y)ₚ et d'un composé ayant une double liaison activée par un groupement carbonyle.

22. Matériau selon la revendication **21**, **caractérisé en ce que** le composé ayant une double liaison activée par un groupement carbonyle est un acrylate, un méthacrylate ou un itaconate de α-alkyl(oligoéthoxy)éthyle de formule générale : 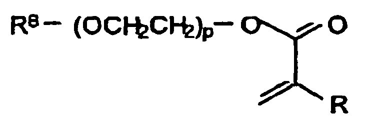
dans laquelle
- p varie entre 2 et 60;
- R représente H, un radical alkyle ou oxa-alkyle de 1 à 12 atomes de carbone, CN ou CH₂COOR¹ dans lequel R¹ est H ou un radical alkyle ou oxa-alkyle de 1 à 12 atomes de carbone; et
- R⁶ est un radical alkyle monovalent de 1 à 18 atomes de carbone, ou un radical aryle monovalent de 5 à 18 atomes de carbone.

23. Matériau selon la revendication **21, caractérisé en ce que** le monomère (A)ₙQ(Y)ₚ est choisi parmi: 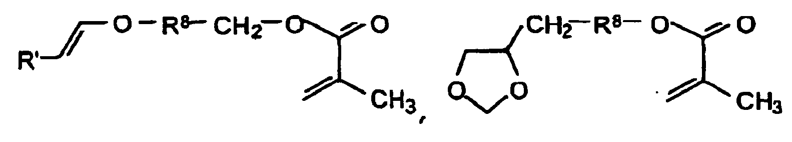 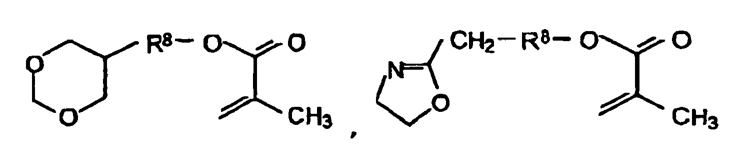
dans lesquels R' représente H ou un radical alkyle de 1 à 12 atomes de carbone, et R⁸ représente un radical alkyle ou oxa-alkyle divalent de 0 à 12 atomes de carbone.

24. Matériau selon la revendication **21, caractérisé en ce qu'**au moins un autre monomère de type acrylate organique ou métallique est utilisé en plus de l'acrylate, du méthacrylate ou de l'itaconate de α-alkyl(oligoéthoxy)-éthyle.

25. Matériau selon la revendication **1**, **caractérisé en ce qu'**il contient un sel de lithium ou un mélange de sel de lithium et d'un sel d'un autre cation.

26. Matériau selon la revendication **1**, **caractérisé en ce** le sel est choisi parmi les sels des anions ClO₄⁻, BF₄⁻, PF₆⁻, AsF₆⁻, SbF₄⁻, R_{f}SO₃⁻, (RₓSO₂NSO₂R'ₓ)⁻, (RₓSO₂C(SO₂R'ₓ)Rₓ")⁻, des anions dérivés du cyclopentadiène et de ses analogues aza portant des groupements électroattracteurs, des anions dérivés de pyrimidine-trione ou de la 1,3-dioxane-4,5-dione portant des groupements électroattracteurs, des dérivés du malononitrile, Rₓ et Rₓ' étant des groupements identiques ou différents dont au moins un possède des atomes électronégatifs, et dans lequel Rₓ" est Rₓ, RₓSO₂- ou Rₓ'SO₂⁻.

27. Matériau selon la revendication **26**, **caractérisé en ce qu'**il contient au moins un sel de ClO₄⁻, BF₄⁻, PF₆⁻, CF₃SO₃⁻, (FSO₂)₂N⁻, (CF₃SO₂)₂N⁻, (C₂F₅SO₂)₂N⁻, [(CF₃SO₂)₂CH]⁻, [(CF₃SO₂)C (CN)₂]⁻, [(CF₃SO₂)₃C]⁻, (CF₃SO₂) NSO₂N (R⁹)₂)⁻ où R⁹ représente alkyle de 1 à 30 atomes de carbone, des anions dérivés du 4,5-dicyano-1,2,3-triazole, du 3-5-bistrifluorométhyl-1,2,4-triazole, ou du tricyanométhane.

28. Matériau selon la revendication **1**, **caractérisé en ce qu'**au moins une fraction des anions est soit portée par la chaîne du polymère, soit fait partie d'une chaîne polymère différente, en mélange ou formant un réseau interpénétré avec le polymère.

29. Matériau selon la revendication **1**, **caractérisé en ce que** les extrémités de chaînes obtenues après polymérisation anionique réagissent au cours de l'activation des groupements Y pour obtenir la réticulation ou co-réticulation pour donner des liens stables de type acétal.

30. Matériau selon la revendication **1 caractérisé en ce que** la masse des polymères avant réticulation est comprise entre 2 x 10³ et 60 x 10³ g/mol.

31. Cellule électrochimique dont l'électrolyte est constitué au moins en partie par un matériau selon la revendication **1**.

32. Système de stockage de l'énergie électrique de type générateur primaire ou secondaire ou super-capacité comprenant au moins une électrode négative et au moins une électrode positive qui comprend au moins en partie un matériau selon l'une quelconque des revendications **1** à **30**.

33. Système selon la revendication **32, caractérisé en ce que** les ions participant aux réactions d'électrodes sont les ions lithium.

34. Système selon la revendication **32, caractérisé en ce que** l'électrode négative comprend le lithium métallique, un des ses alliages, optionnellement sous forme de dispersion nanométrique dans l'oxyde de lithium, les nitrures doubles de lithium et d'un métal de transition, les oxydes à bas potentiel de formule générale Li_{1+y}Ti_{2-x/4}O₄, où x ≥ 0 et y ≤ 1, MoO₂, WO₂, le carbone et les produits carbonés issus de la pyrolyse de matières organiques, les alliages lithium-aluminium ou lithium-silicium.

35. Système selon la revendication **32, caractérisé en ce que** l'électrode positive comprend en outre un autre matériau d'électrode comprenant les oxydes de vanadium Li_{y}VOₓ dans lequel (2x-5 ≤ y ≤ 2x-3; 2.15 ≤ x ≤ 2.5), Li_{y}N_{1-x-z}CoₓAl_{z}O₂ où 0 ≤ x + y ≤ 1 et 0 ≤ y ≤ 1, les spinelles de manganèse Li_{y}Mn₂₋ₓMₓO₄ où M est Li, Cr, Al, v, Ni, 0 ≤ x ≤ 0,5 et 0 ≤ y ≤ 2, les polydisulfures organiques, les polyquinones, FeS, FeS₂, le sulfate de fer, les phosphates et phosphosilicates de fer et de lithium de structure olivine ou Nasicon, ou les produits de substitution du fer par le manganèse, utilisés seuls ou en mélange.

36. Système selon la revendication **32, caractérisé en ce que** le matériau comprend un plastifiant comprenant les liquides polaires choisis parmi le carbonates cycliques et acycliques, la γ-butyrolactone, les esters d'acides carboxylique, les tétraalkylsulfamides, les éthers dialkylés des mono, di, tri et tétraéthylene glycols, les oligomères de masses inférieures à 2000 g/mol, et leurs mélanges.

37. Système de modulation de la lumière comprenant deux supports, dont au moins un transparent, recouverts d'un conducteur à base d'oxyde d'étain ou de d'oxyde d'indium dopé et d'une couche de matériau électrochrome et d'une contre-électrode, **caractérisé en ce que** l'électrolyte est un matériau selon l'une quelconque des revendications **1** à **30.**

38. Système selon la revendication **37,** comprenant en plus du matériau, au moins un colorant susceptible de changer de coloration selon son degré d'oxydation.

## Patentansprüche

1. Ionenleitendes Material, bestehend aus zumindest einem Polymer und zumindest einer ionischen Verbindung, **dadurch gekennzeichnet, dass**:
- das Polymer ein vernetzbares Polymer ist, hergestellt durch anionische Polymerisation eines Monomers der allgemeinen Formel (A)ₙQ(Y)ₚ, gegebenenfalls mit einem oder mehreren anderen Monomeren, worin:
* Q für eine Bindung oder einen organischen Rest der Wertigkeit n+p steht, der gegenüber die anionische oder kationische Polymerisation initiierenden Mitteln nicht reaktiv ist und einen Alkyl-, Alkylaryl- oder Arylalkylrest umfasst, der gegebenenfalls Oxa- oder Aza-Substituenten umfasst und 1 bis 30 Kohlenstoffatome aufweist,
* A für einen bei anionischer Polymerisation reaktiven Rest steht,
* Y ein bei kationischer Polymerisation reaktiver und gegenüber einem Mittel, das anionische Polymerisation initiiert, nicht reaktiver Rest ist und die vernetzbare Funktionalität des vernetzbaren Polymers darstellt,
* n zwischen 1 und 3 variiert,
* p zwischen 1 und 6 variiert; und
- die ionische Verbindung aus zumindest einem Salz Mⁿ⁺Xₙ⁻ besteht, worin Mⁿ⁺ für ein beliebiges anorganisches, organisches oder metallorganisches Kation mit der Ladung n steht und X⁻ ein einwertiges Anion ist, wobei zwei oder mehrere Anionen X-über kovalente Ketten miteinander verbunden sein oder der Kette eines Polymers angehören können.

2. Material nach Anspruch 1, worin:
A zumindest eine der folgenden Gruppen umfasst: 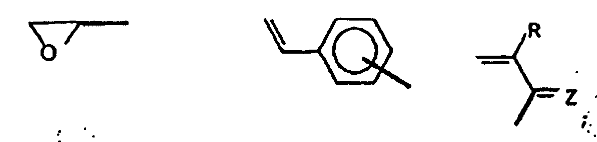 und
Y zumindest eine der folgenden Gruppen umfasst: 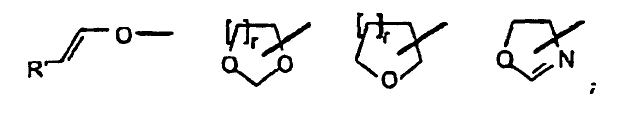
worin:
- Z für O oder CH₂ steht;
- R für H, einen Alkyl- oder Oxaalkylrest mit 1 bis 12 Kohlenstoffatomen, CN oder CH₂COOR¹ steht, worin R¹ H oder ein Alkyl- oder Oxaalkylrest mit 1 bis 12 Kohlenstoffatomen ist;
- R' für H oder einen Alkylrest mit 1 bis 12 Kohlenstoffatomen steht; und
- r zwischen 1 und 6 variiert.

3. Material nach Anspruch 1, **dadurch gekennzeichnet, dass** das Polymer ein statistisches Copolymer, ein Block-Copolymer oder ein Stern-Copolymer ist, das Einheiten, die von einem Monomer (A)ₙQ(Y)ₚ herrühren, und Einheiten, die von einem oder mehreren anderen Monomeren herrühren, aufweist.

4. Material nach Anspruch 1, **dadurch gekennzeichnet, dass** die Formgebung des vernetzbaren Polymers vor der Vernetzung erfolgt.

5. Material nach einem der Ansprüche 1 bis 4, **dadurch gekennzeichnet, dass** das Polymer durch kationische Polymerisation der Gruppierungen Y vernetzt wird.

6. Material nach Anspruch 5, **dadurch gekennzeichnet, dass** die Polymerisation der Gruppierungen Y in Gegenwart von anderen Monomeren, Oligomeren oder Polymeren erfolgt, die Funktionalitäten tragen, die durch die kationische, radikalische oder beide Polymerisationen initiierenden Mittel aktiviert werden können.

7. Material nach Anspruch 6, **dadurch gekennzeichnet, dass** das Monomer aus oxygenierten Derivaten von Dioxolanen vom Spiroorthoformiat- oder Spiroorthocarbonat-Typ ausgewählt ist.

8. Material nach Anspruch 5, **dadurch gekennzeichnet, dass** die Polymerisation der Gruppierungen Y und jedes optionalen Additivs in Monomer-, Oligomer- oder Polymer-Form thermisch, durch Einwirkung aktinischer Strahlung oder durch beides, in Gegenwart eines Initiators oder ohne Initiator, erfolgt.

9. Material nach einem der Ansprüche 1 bis 8, **dadurch gekennzeichnet, dass** es zumindest einen Weichmacher enthält.

10. Material nach Anspruch 5, **dadurch gekennzeichnet, dass** darin ein Additiv in Form von Pulvern, Fasern oder beiden eingebunden wird, das gegebenenfalls so behandelt ist, dass seine Oberfläche erhöhte Verträglichkeit mit und Haftung an dem Polymer oder Polymergemischen aufweist.

11. Material nach Anspruch 10, **dadurch gekennzeichnet, dass** das Additiv organisch oder anorganisch ist und gegebenenfalls die Eigenschaften von Ionen- oder Elektronen-Eigenleitung, optische Eigenschaften, eine erhöhte Dielektrizitätskonstante, piezoelektrische Eigenschaften oder eine Kombination dieser Eigenschaften besitzt.

12. Material nach Anspruch 1, **dadurch gekennzeichnet, dass** das Polymer ein Homopolymer einer Verbindung (A)ₙQ(Y)ₚ, worin A die Gruppe 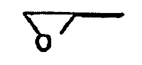 umfasst, oder ein Copolymer einer solchen Verbindung mit einem Epoxid ist.

13. Material nach Anspruch 12, **dadurch gekennzeichnet, dass** das Epoxid Ethylenoxid ist.

14. Material nach Anspruch 12, **dadurch gekennzeichnet, dass** das Monomer (A)ₙQ(Y)ₚ aus den Verbindungen: 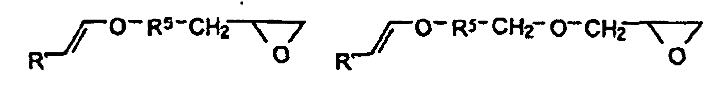 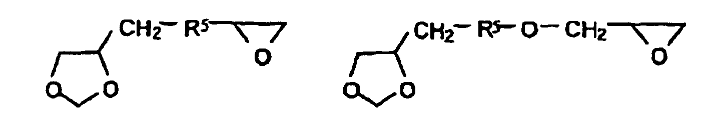 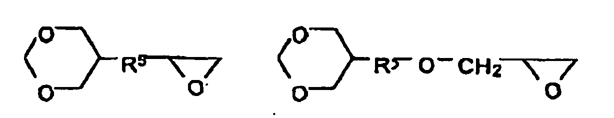 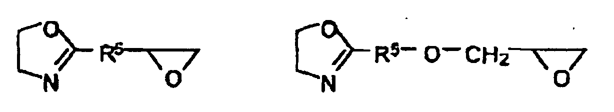 ausgewählt ist, worin:
- R⁵ für einen zweiwertigen Alkyl- oder Oxaalkylrest mit 0 bis 12 Kohlenstoffatomen steht; und
- R' für H oder einen Alkylrest mit 1 bis 12 Kohlenstoffatomen steht.

15. Material nach Anspruch 13, **dadurch gekennzeichnet, dass** zumindest ein Comonomer zusätzlich zu Ethylenoxid verwendet wird.

16. Material nach Anspruch 15, **dadurch gekennzeichnet, dass** das Comonomer aus Propylenoxid, Butylenoxid, Monoepoxiden von Dienen mit 4 bis 10 Kohlenstoffatomen und Glycidolethern mit den Gruppen Methyl, Ethyl oder Allyl ausgewählt ist.

17. Material nach Anspruch 1, **dadurch gekennzeichnet, dass** das vernetzbare Polymer ein Homopolymer einer Verbindung (A)ₙQ(Y)ₚ, worin A die Gruppe 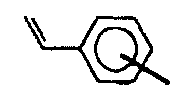 umfasst, oder ein Copolymer einer solchen Verbindung mit einem Styrol-Monomer ist.

18. Material nach Anspruch 17, **dadurch gekennzeichnet, dass** das Styrol-Monomer α-Alkyl(oligoethoxy)-ω-vinylbenzyl der allgemeinen Formel 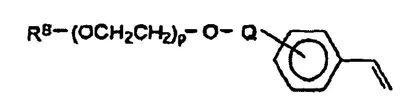 ist, worin:
- p zwischen 2 und 60 variiert;
- R⁵ ein einwertiger Alkylrest mit 1 bis 18 Kohlenstoffatomen oder ein einwertiger Arylrest mit 5 bis 18 Kohlenstoffatomen ist;
- Q für (CH₂)_{q}, -CO- oder -SO₂- steht; und
- q zwischen 0 und 4 variiert.

19. Material nach Anspruch 17, **dadurch gekennzeichnet, dass** das Monomer (A)ₙQ(Y)ₚ ausgewählt ist aus: 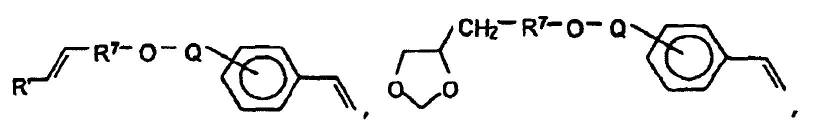 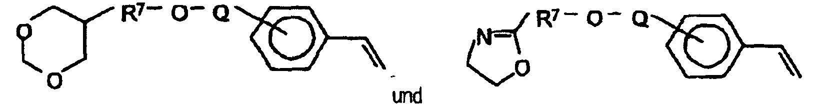
worin R' für H oder einen Alkylrest mit 1 bis 12 Kohlenstoffatomen steht, Q für (CH₂)_{q}, -CO- oder -SO₂- steht und R⁷ gleich R⁵ ist.

20. Material nach Anspruch 18, **dadurch gekennzeichnet, dass** zumindest ein anderes Monomer zusätzlich zu α-Alkyl(oligoethoxy)-ω-vinylbenzyl verwendet wird.

21. Material nach Anspruch 1, **dadurch gekennzeichnet, dass** das vernetzbare Polymer ein Homopolymer einer Verbindung (A)ₙQ(Y)ₚ, worin A eine Gruppe 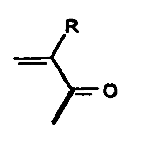 umfasst, worin R für H, einen Alkyl- oder Oxaalkylrest mit 1 bis 12 Kohlenstoffatomen, CN oder CH₂COOR¹ steht, worin R¹ H oder ein Alkyl- oder Oxaalkylrest mit 1 bis 12 Kohlenstoffatomen ist, oder ein Copolymer einer solchen Verbindung (A)ₙQ(Y)ₚ mit einer Verbindung mit einer durch eine Carbonylgruppe aktivierten Doppelbindung ist.

22. Material nach Anspruch 21, **dadurch gekennzeichnet, dass** die Verbindung mit einer durch eine Carbonylgruppierung aktivierten Doppelbindung ein Acrylat, ein Methacrylat oder ein Itaconat von α-Alkyl(oligoethoxy)ethyl der allgemeinen Formel: 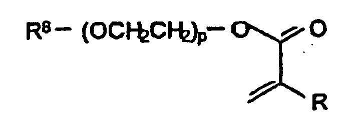 ist, worin:
- p zwischen 2 und 60 variiert;
- R für H, einen Alkyl- oder Oxaalkylrest mit 1 bis 12 Kohlenstoffatomen, CN oder CH₂COOR¹ steht, worin R¹ H oder ein Alkyl- oder Oxaalkylrest mit 1 bis 12 Kohlenstoffatomen ist; und
- R⁶ ein einwertiger Alkylrest mit 1 bis 18 Kohlenstoffatomen oder ein einwertiger Arylrest mit 5 bis 18 Kohlenstoffatomen ist.

23. Material nach Anspruch 21, **dadurch gekennzeichnet, dass** das Monomer (A)ₙQ(Y)ₚ ausgewählt ist aus: 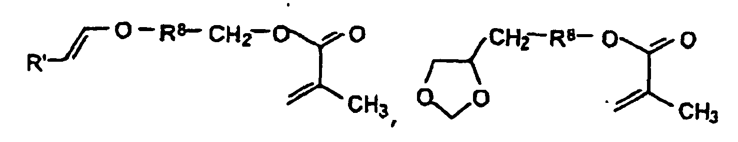 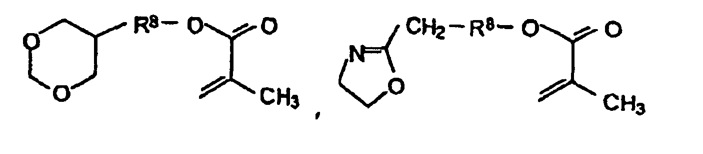
worin R' für H oder einen Alkylrest mit 1 bis 12 Kohlenstoffatomen steht und R⁸ für einen zweiwertigen Alkyl- oder Oxaalkylrest mit 0 bis 12 Kohlenstoffatomen steht.

24. Material nach Anspruch 21, **dadurch gekennzeichnet, dass** zumindest ein anderes Monomer vom Typ organisches oder Metallacrylat zusätzlich zum Acrylat, Methacrylat oder Itaconat von α-Alkyl(oligoethoxy)ethyl verwendet wird.

25. Material nach Anspruch 1, **dadurch gekennzeichnet, dass** es ein Lithiumsalz oder ein Gemisch aus Lithiumsalz und einem Salz eines anderen Kations enthält.

26. Material nach Anspruch 1, **dadurch gekennzeichnet, dass** das Salz aus Salzen der Anionen ClO₄⁻, BF₄⁻, PF₆⁻, AsF₆⁻, SbF₄⁻, R_{f}SO₃⁻, (RₓSO₂NSO₂R'ₓ)⁻, (RₓSO₂C(SO₂R'ₓ)Rₓ")⁻, der von Cyclopentadien und seinen Aza-Analogen, die elektronenanziehende Gruppen tragen, abgeleiteten Anionen, der von Pyrimidintrion oder 1,3-Dioxan-4,5-dion, die elektronenanziehende Gruppierungen tragen, abgeleiteten Anionen und von Malonsäuredinitril-Derivaten ausgewählt ist, worin Rₓ und Rₓ' gleiche oder unterschiedliche Gruppierungen sind, von denen zumindest eine elektronegative Atome aufweist, und worin Rₓ" für Rₓ, RₓSO₂- oder Rₓ'SO₂⁻ steht.

27. Material nach Anspruch 26, **dadurch gekennzeichnet, dass** es zumindest ein Salz von ClO₄⁻, BF₄⁻, PF₆⁻, CF₃SO₃⁻, (FSO₂)₂N⁻, (CF₃SO₂)₂N⁻, (C₂F₅SO₂)₂N⁻, [(CF₃SO₂)₂CH]⁻, [(CF₃SO₂)C(CN)₂]⁻, [(CF₃SO₂)₃C]⁻, (CF₃SO₂)NSO₂N(R⁹)₂)⁻, worin R⁹ für Alkyl mit 1 bis 30 Kohlenstoffatomen steht, von von 4,5-Dicyano-1,2,3-triazol, von 3,5-Bistrifluormethyl-1,2,4-triazol oder von Tricyanomethan abgeleiteten Anionen enthält.

28. Material nach Anspruch 1, **dadurch gekennzeichnet, dass** zumindest ein Teil der Anionen entweder von der Polymerkette getragen wird oder Teil einer anderen Polymerkette ist, die mit dem Polymer ein Gemisch oder ein interpenetrierendes Netzwerk bildet.

29. Material nach Anspruch 1, **dadurch gekennzeichnet, dass** die Enden der nach anionischer Polymerisation erhaltenen Ketten im Laufe der Aktivierung der Gruppierungen Y reagieren, um Vernetzung oder Co-Vernetzung zu stabilen Verbindungen vom Acetaltyp zu erzielen.

30. Material nach Anspruch 1, **dadurch gekennzeichnet, dass** die Masse der Polymere vor der Vernetzung im Bereich zwischen 2 x 10³ und 60 x 10³ g/mol beträgt.

31. Elektrochemische Zelle, deren Elektrolyt zumindest zu einem Teil aus einem Material nach Anspruch 1 besteht.

32. Speichersystem für elektrische Energie vom Typ einer Primär- oder Sekundärzelle oder einer Superkapazität, umfassend zumindest eine negative Elektrode und zumindest eine positive Elektrode, die zumindest zu einem Teil ein Material nach einem der Ansprüche 1 bis 30 umfasst.

33. System nach Anspruch 32, **dadurch gekennzeichnet, dass** die an den Elektrodenreaktionen beteiligten Ionen Lithiumionen sind.

34. System nach Anspruch 32, **dadurch gekennzeichnet, dass** die negative Elektrode metallisches Lithium, eine Legierung davon, gegebenenfalls in Form einer nanometrischen Dispersion in Lithiumoxid, Doppelnitride von Lithium und einem Übergangsmetall, Oxide mit niedrigem Potenzial der allgemeinen Formel Li_{1+y}Ti_{2-x/4}O₄, worin x ≥ 0 ist und y ≤ 1 ist, MoO₂, WO₂, Kohlenstoff und kohlenstoffhältige Produkte der Pyrolyse organischer Stoffe, Lithium-Aluminium- oder Lithium-Silicium-Legierungen umfasst.

35. System nach Anspruch 32, **dadurch gekennzeichnet, dass** die positive Elektrode zusätzlich ein anderes Elektrodenmaterial umfasst, das Vanadiumoxide Li_{y}VOₓ, worin gilt: 2x-5 ≤ y ≤ 2x-3 und 2,15 ≤ x ≤ 2,5, Li_{y}N₁₋ₓ₋₂CoₓAl₂O₂, worin gilt: 0 ≤ x+y ≤ 1 und 0 ≤ y ≤ 1, Manganspinelle Li_{y}Mn_{2-y}MₓO₄, worin M für Li, Cr, Al, V, Ni steht und gilt: 0 ≤ x ≤ 0,5 und 0 ≤ y ≤ 2, organische Polydisulfide, Polychinone, FeS, FeS₂, Eisensulfat, Eisen- und Lithiumphosphate und -phosphosilicate mit Olivin- oder Nasicon-Struktur oder Produkte der Ersetzung von Eisen durch Mangan, alleine oder als Gemische eingesetzt, umfassend.

36. System nach Anspruch 32, **dadurch gekennzeichnet, dass** das Material einen Weichmacher umfasst, der polare Flüssigkeiten, ausgewählt aus zyklischen und azyklischen Carbonaten, γ-Butyrolacton, Carbonsäureestern, Tetraalkytsulfamiden, Dialkylethern von Mono-, Di-, Tri- und Tetraethylenglykolen, Oligomere mit Massen unter 2.000 g/mol und Gemische daraus umfasst.

37. System zur Lichtmodulation, umfassend zwei Träger, von denen zumindest einer transparent ist, bedeckt mit einem auf Zinn- oder Indiumoxidbasis dotierten Leiter und einer Schicht aus elektrochromen Material, sowie eine Gegenelektrode, **dadurch gekennzeichnet, dass** der Elektrolyt ein Material nach einem der Ansprüche 1 bis 30 ist.

38. System nach Anspruch 37, umfassend zusätzlich zum Material zumindest einen Farbstoff, der in der Lage ist, je nach Oxidationsgrad seine Farbe zu ändern.

## Claims

1. Ionic conduction material comprising at least one polymer and at least one ionic compound, **characterized in that**
- the polymer is a crosslinkable polymer obtained by anionic polymerization of a monomer having the general formula (A)ₙQ(Y)ₚ and optionally with one or more other monomers, wherein
* Q represents a bond or an organic radical of n + p valence which is non reactive towards reagents initiating anionic or cationic polymerization, said organic radical being selected from alkyl, alkylaryl, arylalkyl, optionally comprising oxa or aza substituents, and comprising from 1 to 30 carbon atoms,
* A represents a radical which is reactive in anionic polymerization,
* Y is a radical which is reactive in cationic polymerization and non-reactive towards agents initiating anionic polymerization, and represents the crosslinkable group of said crosslinkable polymer,
* n varies between 1 and 3; and
* p varies between 1 and 6, and
* the ionic compound comprises at least one salt Mⁿ⁺Xₙ⁻wherein Mⁿ⁺ is an inorganic, organic or organometallic cation of charge n, and X⁻ is a monovalent anion, and wherein two or more X⁻ can be linked together with covalent chains or chains belonging to a polymer chain.

2. A material according to claim 1, wherein comprises at least one of the following groups 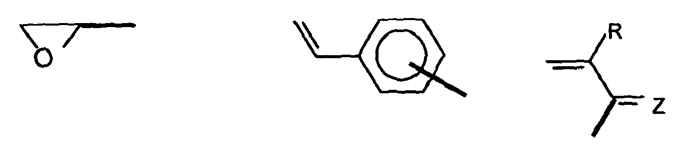 Y comprises at least one of the following groups 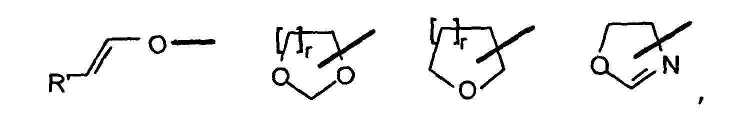
**characterized in that** :
- Z represents O or CH₂;
- R represents H, an alkyl or oxa-alkyl radical having 1 to 12 carbon atoms, CN or CH₂COOR¹ wherein R¹ is H or an alkyl or oxa-alkyl radical having 1 to 12 carbon atoms ;
- R' represents H or an alkyl radical having 1 to 12 carbon atoms ; and
- r varies between 1 and 6.

3. A material according to claim 1, **characterized in that** the polymer is a statistical copolymer, a bloc copolymer or a star polymer having units derived from a (A)ₙQ(Y)ₚ monomer and units derived from one or more other monomers.

4. A material according to claim 1, **characterized in that** the molding of the crosslinkable polymer is carried out prior to the crosslinking.

5. A material according to one of claims 1 to 4, **characterized in that** the polymer is crosslinked via cationic polymerization of the Y groups.

6. A material according to claim 5, **characterized in that** the polymerization of the Y groups is carried out in the presence of other monomers, oligomers or polymers bearing functions that can be activated by a reagent initiating cationic polymerization, radicalar polymerization, or both.

7. A material according to claim 6, **characterized in that** the monomer is selected from the oxygenated derivatives of the dioxolanes of spiroorthoformate or spiroorthocarbonate type.

8. A material according to claim 5, **characterized in that** the polymerization of the Y groups and of any optional additives in the form of monomers, oligomers or polymers is carried out thermally, through actinic radiation, or both, optionally in the presence of initiators or sensitizers.

9. A material according to any of claim 1 to 9, **characterized in that** it comprises at least one plasticizer.

10. A material according to claim 5, **characterized in that** it comprises an additive in the form of powders, fibers, or both, optionally treated to have a surface showing enhanced compatibility and adhesion toward the polymer or the mixture of polymers.

11. A material according to claim 10, **characterized in that** said additive is organic or inorganic, and has intrinsic ionic or electronic conduction properties, optical properties, a high dielectric constant, piezoelectric properties, or combinations thereof.

12. A material according to claim 1, **characterized in that** said polymer is an homopolymer of a coumpound (A)ₙQ(Y)ₚ wherein A comprises a group 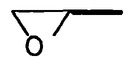 or a copolymer of such a compound and an epoxide.

13. A material according to claim 12, **characterized in that** the epoxide is ethylene oxide.

14. A material according to claim 12, **characterized in that** the monomer (A)ₙQ(Y)ₚ is selected from : 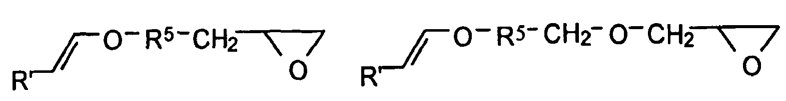 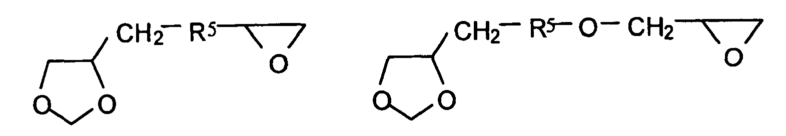 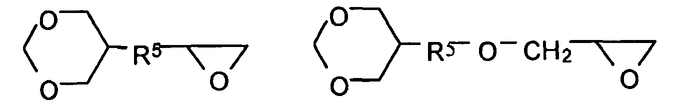 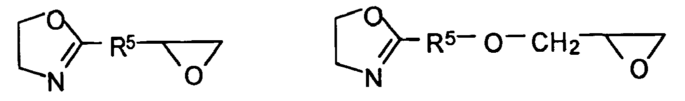
wherein
- R⁵ is a divalent alkyl or oxa-alkyl radical having from 0 to 12 carbon atoms; and
- R' is H or an alkyl radical having 1 to 12 carbon atoms.

15. A material according to claim 13 **characterized in that** at least one co-monomer is used in addition to ethylene oxide.

16. A material according to claim 15 **characterized in that** the co-monomer comprises propylene oxide, butylene oxide, monoepoxide of dienes having from 4 to 10 carbon atoms, glycidol ethers with methyl, ethyl and allyl groups.

17. A material according to claim 1, **characterized in that** the crosslinkable polymer is an homopolymer of a compound (A)ₙQ(Y)ₚ wherein A comprises a group 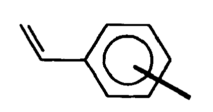 or a copolymer of such a compound and of a styrenic monomer.

18. A material according to claim 17, **characterized in that** said said styrenic monomer is α-alkyl(oligoethoxy)-ω-vinylbenzyl of general formula : 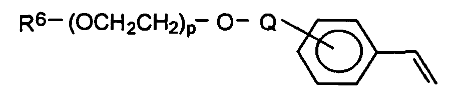
wherein
- p varies between 2 and 60;
- R⁶ is a monovalent alkyl radical having from 1 to 18 carbon atoms, or a monovalent aryl radical having from 5 to 18 carbon atoms;
- Q is (CH₂)_{q}, -CO- or -SO₂- ; and
- q varies between 0 and 4.

19. A material according to claim 17, **characterized in that** the monomer (A)ₙQ(Y)ₚ is selected from 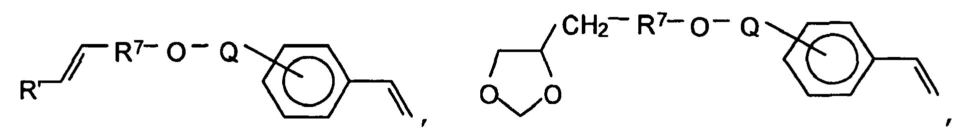 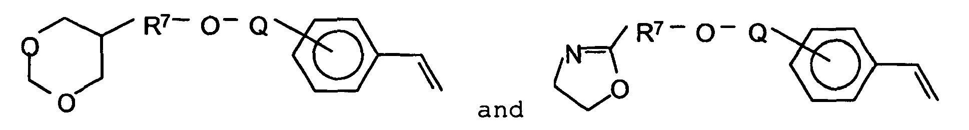
wherein R' is H or an alkyl radical having from 1 to 12 carbon atoms, Q is (CH₂)_{q}, -CO- or -SO₂- and R⁷ is R⁵.

20. A material according to claim 18, **characterized in that** at least one other monomer is used in addition to α-alkyl(oligoethoxy)-ω-vinylbenzyl.

21. A material according to claim 1, **characterized in that** the crosslinkable polymer is an homopolymer of a compound (A)ₙQ(Y)ₚ wherein A comprises 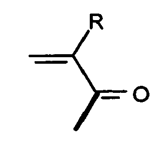
wherein R represents H, and alkyl or oxa-alkyl radical of 1 to 12 carbon atoms, CN or CH₂COOR¹ wherein R¹ is H or an alkyl or oxa-alkyl radical of 1 to 12 carbon atoms, or a copolymer of a monomer (A)ₙQ(Y)ₚ and of a compound having a double bond activated by a carbonyl group.

22. A material according to claim 21, **characterized in that** the compound having a double bond activated by a carbonyl group is an α-alkyl(oligoethoxy)ethyl acrylate, methacrylate or itaconate, of general formula : 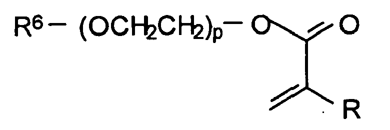
wherein
- p varies between 2 and 60;
- R represents H, an alkyl or oxa-alkyl radical having 1 to 12 carbon atoms, CN or CH₂COOR¹ wherein R¹ is H or an alkyl or oxa-alkyl radical having 1 to 12 carbon atoms;
- R⁶ is a monovalent alkyl radical having from 1 to 18 carbon atoms or a monovalent aryl ra dical having from 5 to 18 carbon atoms.

23. A material according to claim 21, **characterized in that** the monomer (A)ₙQ(Y)ₚ is selected from 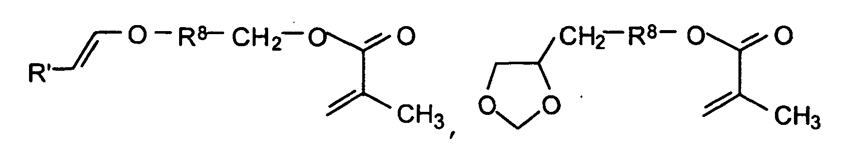 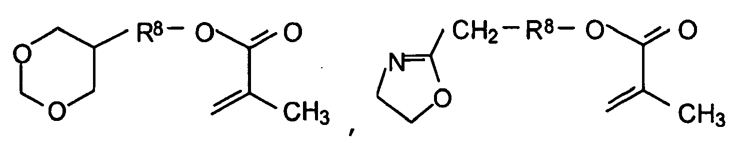
wherein R' is a divalent alkyl or oxa-alkyl radical having 0 to 12 carbon atoms, R represents H, an alkyl or oxa-alkyl radical having from 1 to 12 carbon atoms, CN or CH₂COOR', wherein R' is H or an alkyl or oxa-alkyl radical having 1 to 12 carbon atoms.

24. A material according to claim 21, **characterized in that** at least one other monomer comprising an organic or metallic acrylate is used in addition to α-alkyl(oligoethoxy)ethyl acrylate, methacrylate or itaconate.

25. A material according to claim 1, **characterized in that** it contains a lithium salt or a mixture of a lithium salt and a salt of another cation.

26. A material according to claim 1, **characterized in that** the salt is selected from salts of an anion selected from ClO₄⁻, BF₄⁻, PF₆⁻, AsF₆⁻, SbF₄⁻, R_{f}SO₃⁻, (RₓSO₂NSO₂R'ₓ)⁻, (RₓSO₂C(SO₂R'ₓ)Rₓ")⁻, anions derived from cyclopentadiene and aza analogs thereof bearing electroattracting groups, from anions derived from pyrimidine-trione or 1,3-dioxane-4,5-dione bearing electroattracting groups, and from malononitrile derivatives, wherein Rₓ and Rₓ' are the same or different and at least one has electronegative atoms, and wherein Rₓ" is Rₓ, RₓSO₂- or Rₓ'SO₂⁻.

27. A material according to claim 26, **characterized in that** it contains at least one salt of an anion selected from ClO₄⁻, BF₄⁻, PF₆⁻, CF₃SO₃⁻, (FSO₂)₂N⁻, (CF₃SO₂)₂N⁻, (C₂F₅SO₂)₂N⁻, [(CF₃SO₂)₂CH]⁻, [(CF₃SO₂)C(CN)₂]⁻, [(CF₃SO₂)₃C]⁻, (CF₃SO₂)NSO₂N(R⁹)₂)⁻ wherein R⁹ represents an alkyl of from 1 to 30 carbon atoms, or from anions derived from 4,5-dicyano-1,2,3-triazole, 3-5-bistrifluoromethyl-1,2,4-triazole, or tricyanomethane.

28. A material according to claim 1, **characterized in that** at least a fraction of the anions is borne by the chain of the polymer or is part of the chain of a different polymer, in a mixture or forming an interpenetrated network with the polymer.

29. A material according to claim 1, **characterized in that** the ends of the chains obtained through anionic polymerization react during activation of the Y groups to cause crosslinking or co-crosslinking which provides stable acetal bonds.

30. A material according to claim 1, **characterized in that** the polymer weight before cross-linking is comprised between 2 x 10³ and 60 x 10³ g/mol.

31. An electrochemical cell wherein the electrolyte comprises at least in part a material according to claim 1.

32. An electrical energy storage system of the primary or secondary generator type, or super-capacitor, comprising at least one negative electrode and at least one positive electrode comprising at least in part a material according to claims 1 to 30.

33. A system according to claim 32, **characterized in that** the ions participating to the electrode reactions are lithium ions.

34. A system according to claim 32 **characterized in that** the negative electrode comprises metallic lithium or an alloy thereof, optionally in the form of a nanometric dispersion in lithium oxide, double nitrides of lithium and a transition metal, low potential oxides of general formula Li_{1+y}Ti_{2-x/4}O₄, wherein x ≥ 0 and y ≤ 1, MoO₂, WO₂, carbon and carbonated products obtained from organic material pyrolysis, lithium-aluminum or lithium-silicon alloys.

35. A system according to claim 32, **characterized in that** the positive electrode further comprises another material comprising vanadium oxides Li_{y}VOₓ wherein (2x-5 ≤ y ≤ 2x-3; 2,15 ≤ x ≤ 2,5), Li_{y}N_{1-x-z}CoₓAl_{z}O₂ wherein 0 ≤ x + y ≤ 1 and 0 ≤ y ≤ 1, manganese spinels Li_{y}Mn₂₋ₓMₓO₄ wherein M is Li, Cr, Al, V, Ni, 0 ≤ x ≤ 0,5 and 0 ≤ y ≤ 2, organic polydisulfides, polyquinones, FeS, FeS₂, iron sulphate, phosphates and phosphosilicates of iron and lithium of the olivine or Nasicon-type structure, or substitution products of iron with manganese, used alone or in combination.

36. A system according to claim 32, **characterized in that** the material further comprises a plasticizer comprising polar liquids selected from acyclic and cyclic carbonates, γ-butyrolactone, carboxylic acid esters, tetraalkylsulfamides, dialkylated ethers of mono, di, tri and tetraethylene glycols, and oligomers of a weight lower than 2000 g/mol, and mixtures thereof.

37. System for light modulation comprising two supports wherein at least one is transparent, coated with a tin oxide based conductor or doped indium oxide conductor, and a layer of an electrochromic material and a counterelectrode **characterized in that** the electrolyte is a material according to claims 1 to 30.

38. A system according to claim 37 further comprising a coloring agent susceptible of changing of coloration depending on its degree of oxidation.
